(19) Europäisches Patentamt
European Patent Office
Office européen des brevets

(11) Veröffentlichungsnummer: **0 319 689 B1**

(12) # EUROPÄISCHE PATENTSCHRIFT

(45) Veröffentlichungstag der Patentschrift: **15.12.93**

(51) Int. Cl.5: **C07D 417/12**, A01N 47/36

(21) Anmeldenummer: **88117652.3**

(22) Anmeldetag: **24.10.88**

Die Akte enthält technische Angaben, die nach dem Eingang der Anmeldung eingereicht wurden und die nicht in dieser Patentschrift enthalten sind.

(54) **Heterocyclisch substituierte N-Sultam-sulfonamide, Verfahren zu ihrer Herstellung und ihre Verwendung als Herbizide und Pflanzenwachstumsregulatoren.**

(30) Priorität: **31.10.87 DE 3736959**

(43) Veröffentlichungstag der Anmeldung:
**14.06.89 Patentblatt 89/24**

(45) Bekanntmachung des Hinweises auf die Patenterteilung:
**15.12.93 Patentblatt 93/50**

(84) Benannte Vertragsstaaten:
**AT BE CH DE ES FR GB GR IT LI NL**

(56) Entgegenhaltungen:
**EP-A- 0 107 979**
**EP-A- 0 131 258**
**EP-A- 0 202 762**

(73) Patentinhaber: **HOECHST AKTIENGESELLSCHAFT**

**D-65926 Frankfurt(DE)**

(72) Erfinder: **Willms, Lothar, Dr.**
**Schulstrasse 3**
**D-5411 Hillscheid(DE)**
Erfinder: **Bauer, Klaus, Dr.**
**Doorner Strasse 53D**
**D-6450 Hanau(DE)**
Erfinder: **Bieringer, Hermann, Dr.**
**Eichenweg 26**
**D-6239 Eppstein/Taunus(DE)**
Erfinder: **Bürstell, Helmut, Dr.**
**Am Hohlacker 65**
**D-6000 Frankfurt am Main 50(DE)**

EP 0 319 689 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist bekannt, daß heterocyclisch substituierte Sulfonylharnstoffe herbizide und pflanzenwachstumsregulierende Eigenschaften aufweisen (z.B. EP-A-131 258).

Diese weisen jedoch bei ihrer Anwendung Nachteile auf, wie beispielsweise eine hohe Persistenz oder unzureichende Selektivität.

Es wurde nun gefunden, daß heterocyclisch substituierte N-Sultam-sulfonamide als Herbizide und Pflanzenwachstumsregulatoren besonders geeignet sind.

Gegenstand der vorliegenden Erfindung sind daher Verbindungen der Formel (I) oder deren Salze

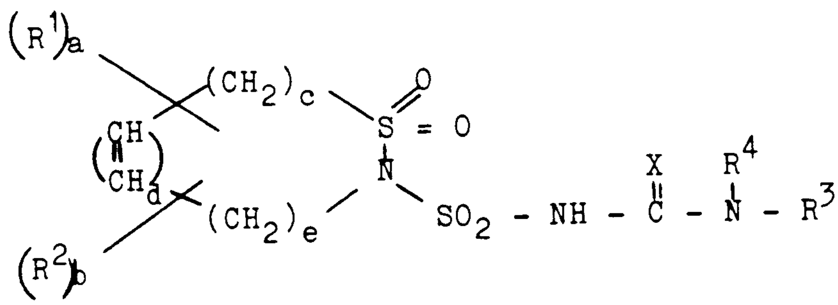
(I),

worin

| | |
|---|---|
| $R^1$ und $R^2$ | unabhängig voneinander Wasserstoff, Halogen, $(C_1$-$C_8)$-Alkyl, $(C_2$-$C_8)$-Alkenyl, $(C_2$-$C_8)$-Alkinyl oder $(C_1$-$C_8)$-Alkoxy, wobei diese Reste gegebenenfalls ein-oder mehrfach durch Halogen oder ein- oder zweifach durch $(C_1$-$C_4)$-Alkoxy oder $(C_1$-$C_4)$-Alkylthio substituiert sein können; $-(CH_2)_n-COOR^{11}$, wobei n eine Zahl zwischen 0 und 2 bedeutet, |
| $R^3$ | Wasserstoff; $(C_1$-$C_8)$-Alkyl; $(C_2$-$C_8)$-Alkenyl oder $(C_2$-$C_8)$-Alkinyl, |
| $R^4$ | einen heterocyclischen Rest der Formeln |

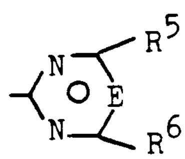
oder
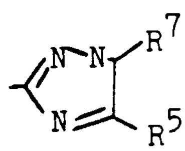

| | |
|---|---|
| | wobei E = CH oder N ist, |
| $R^5$, $R^6$ | unabhängig voneinander Wasserstoff; Halogen; $(C_1$-$C_4)$-Alkyl oder $(C_1$-$C_4)$-Alkoxy, die beide gegebenenfalls ein- oder mehrfach halogeniert sein können; Di-[$(C_1$-$C_4)$-alkoxy]-$(C_1$-$C_2)$-alkyl; $(C_3$-$C_6)$-Cycloalkyl, $-OCHR^8COOR^9$; $-NR^9R^{10}$ oder $(C_1$-$C_4)$-Alkylthio, |
| $R^7$ | $(C_1$-$C_4)$-Alkyl, |
| $R^8$ | Wasserstoff oder $(C_1$-$C_4)$-Alkyl und |
| $R^9$, $R^{10}$ | unabhängig voneinander Wasserstoff; $(C_1$-$C_4)$-Alkyl; $(C_2$-$C_4)$-Alkenyl oder $(C_2$-$C_4)$-Alkinyl |
| $R^{11}$ | Wasserstoff, $(C_1$-$C_8)$-Alkyl, $(C_2$-$C_4)$-Alkenyl, $(C_2$-$C_4)$-Alkinyl, welche gegebenenfalls durch Halogen oder $(C_1$-$C_4)$-Alkoxyreste ein- oder mehrfach substituiert sein können, |
| X | Sauerstoff oder Schwefel und |
| a, b, c, d und e | unabhängig voneinander die Zahl 0, 1 oder 2 bedeuten, mit der Maßgabe, daß die Summe von c + d + e größer oder gleich 2 ist. |

Die Verbindungen der Formel I können Salze bilden, bei denen der Wasserstoff der $-SO_2$-NH-Gruppe durch ein für die Landwirtschaft geeignetes Kation ersetzt wird. Diese Salze sind im allgemeinen Metall-, insbesondere Alkali-, Erdalkali-, gegebenenfalls alkylierte Ammonium- oder organische Aminsalze. Sie werden vorzugsweise in inerten Lösungsmitteln wie z.B. Wasser, Methanol oder Aceton bei Temperaturen von 0 bis 100 °C hergestellt. Geeignete Basen zur Herstellung der erfindungsgemäßen Salze sind beispielsweise Alkalicarbonate, wie Kaliumcarbonate, Alkali- und Erdalkalihydroxide, Ammoniak oder Ethanolamin.

Bevorzugte Verbindungen der Formel I sind solche, bei denen $R^1$, $R^2$ unabhängig voneinander Wasserstoff, $(C_1\text{-}C_4)$-Alkyl, das wie oben beschrieben substituiert ist, oder Halogen;a,b,c, d,e, unabhängig voneinander die Zahlen 0, 1 oder 2 bedeuten, jedoch mit der Maßgabe, daß $c + d + e \geqq 2$ und $\leqq 4$ ist, $R^3$ Wasserstoff, $(C_1\text{-}C_4)$-Alkyl oder Allyl; $R^4$ einen Rest der Formel

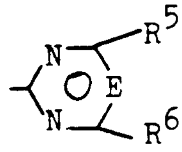

und $R^5$ und $R^6$ unabhängig voneinander Halogen, $(C_1\text{-}C_4)$-Alkyl oder $(C_1\text{-}C_4)$-Alkoxy, die beide durch Halogen substituiert sein können, bedeuten und X für Sauerstoff steht.

Halogen bedeutet vorzugsweise Fluor, Chlor oder Brom. Unter halogeniertem Alkyl oder halogeniertem Alkoxy sind insbesondere zu verstehen die Reste $CF_3$, $CH_2\text{-}CH_2Cl$, $CH_2CH_2Br$, $OCF_2H$, $OCH_2CF_3$. Halogeniertes Alkenyl oder halogeniertes Alkinyl bedeutet insbesondere $CH_2CH=CHCl$, $CH_2CCl=CCl_2$, $CH_2\text{-}C{\equiv}CCH_2\text{-}Cl$. $(C_3\text{-}C_6)$Cycloalkyl bedeutet insbesondere Cyclopropyl. Besonders bevorzugte Verbindungen der Formel (I) sind solche, worin $R^1$ und/oder $R^2$ Wasserstoff, $(C_1\text{-}C_4)$-Alkyl, das wie oben beschrieben substituiert ist, $R^3$ Wasserstoff, $a + b = 0$, 1 oder 2, $d = 0$, $c + e = 3$ oder 4, insbesondere 4, $R^4$ einen Rest der Formel

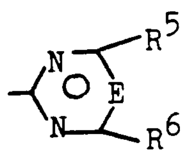

und $R^5$ und $R^6$ unabhängig voneinander Chlor, Brom, $(C_1\text{-}C_4)$-Alkyl, $(C_1\text{-}C_4)$-Alkoxy, $OCF_2H$, $OCH_2CF_3$ oder $CF_3$ bedeuten, sowie deren Salze.

Weiterer Gegenstand der vorliegenden Erfindung sind Verfahren zur Herstellung von Verbindungen der allgemeinen Formel (I), dadurch gekennzeichnet, daß man

(a) eine Verbindung der Formel (II)

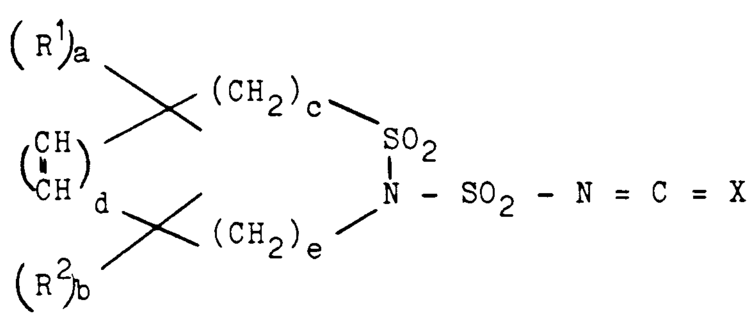

(II)

mit einer Verbindung der Formel (III)

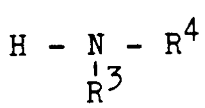

(III)

umsetzt, oder

(b) für X = Sauerstoff eine Verbindung der Formel (IV)

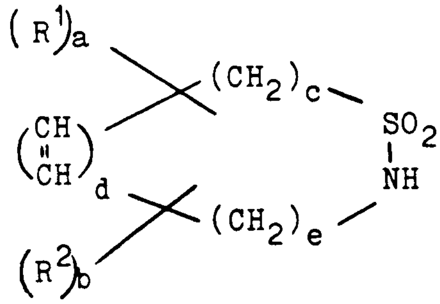

(IV)

mit einem Chlorsulfonylharnstoff der Formel (V)

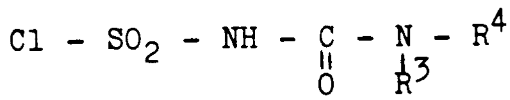

(V)

umsetzt und die erhaltene Verbindung gegebenenfalls in ihr Salz überführt.

Die Umsetzung der Verbindungen der Formel (II) und (III) erfolgt vorzugsweise in inerten aprotischen Lösungsmitteln, wie z.B. Acetonitril, Dichlormethan, Toluol, Tetrahydrofuran oder Dioxan bei Temperaturen zwischen 0 °C und der Siedetemperatur des Lösungsmittels.

Die N-Sultamsulfonylisocyanate der Formel II sind neu und lassen sich durch Umsetzung der Sultame der Formel II mit Chlorsulfonylisocyanat herstellen. Die Verbindungen der Formel II und das Verfahren zu ihrer Herstellung sind daher ebenfalls ein Teil der vorliegenden Erfindung. Dieses Verfahren wird vorzugsweise in inerten organischen Lösungsmitteln wie z.B. Toluol, Xylol oder Chlorbenzol in Anlehnung an die bekannte Umsetzung von offenkettigen sekundären Sulfonamiden mit Chlorsulfonylisocyanat durchgeführt (DE-OS 2,257,240).

Die Umsetzung der Verbindung (IV) mit den Chlorsulfonylharnstoffen (V) führt man vorzugsweise in inerten Lösungsmitteln wie z.B. Dichlormethan, Acetonitril, Tetrahydrofuran, Dioxan oder Dimethoxyethan bei Temperaturen zwischen -70 °C und 150 °C gegebenenfalls in Gegenwart einer Base als HCl-bindendes Agens durch. Als Basen können Alkali- oder Erdalkalicarbonate bzw. -bicarbonate wie z.B. $K_2CO_3$, $NaHCO_3$, $Na_2CO_3$ oder tertiäre Amine wie z.B. Pyridin oder Triethylamin eingesetzt werden.

Die Sultame (V) sind literaturbekannt oder können nach literaturbekannten Verfahren hergestellt werden (s. z.B. Liebigs Ann. 646 (1961), S.32-45; Liebigs Ann. 651 (1962), S. 17-29; C.A. 89 (1978), 179478 Z; Bull. Chem. Soc. Jap. 44 (1971), 771-777, Tetr. Lett. (1972), S.213; Chem. Ber. 93 (1960), S. 784).
Die Chlorsulfonylharnstoffe (V) sind aus Aminen der Formel (III) und Chlorsulfonylisocyanat zugänglich (EP-A 141 199).

Die Ausgangsstoffe der Formel (III) sind bekannt oder lassen sich nach im Prinzip bekannten Verfahren herstellen, z.B. durch Cyclisierung entsprechender Guanidinderivate mit entsprechend substituierten 1,3-Diketonen, vergleiche z.B. "The Chemistry of Heterocyclic Compounds", Vol. XVI (1962) and Supplement I (1970), oder durch Derivatisierung von Cyanurchlorid, vgl. z.B. "The Chemistry of Heterocyclic Compounds", L. Rapaport: "s-Triazines and Derivatives" (1959).

Die erfindungsgemäßen Verbindungen der Formel I weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöckchen oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im Einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria etc. sowie Cyperusarten aus der annuellen Gruppe und auf seiten der perennierenden Spezies Agropyron, Cynodon, Imperata sowie Sorghum etc. und auch ausdauernde Cyperusarten gut erfaßt.

Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon, Sida etc. auf der annuellen Seite sowie Convolvulus, Cirsium, Rumex, Artemisia etc. bei den Perennierenden.

Unter den spezifischen Kulturbedingungen im Reis vorkommende Unkräuter wie z.B. Sagittaria, Alisma, Eleocharis, Scirpus, Cyperus etc. werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wuchsstadium stehen oder sterben nach einer gewissen Zeit mehr oder weniger schnell ab, so daß auf diese Weise eine für die Kulturpflanzen schädliche Unkrautkonkurrenz sehr früh und nachhaltig durch den Einsatz der neuen erfindungsgemäßen Verbindungen beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrüben, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z.B. durch Auslösen von Desikkation, Abszission und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel, z.B. ligninsulfonsaures Natrium, 2,2′-dinaphthylmethan-6,6′-disulfonsaures Natrium, dibutylnaphthalinsulfonsaures Natrium oder auch oleoylmethyltaurinsures Natrium enthalten. Die Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze wie Ca-dodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyocethylensorbitanfettsäureester oder Polyoxethylensorbitester.

Stäubemittel kann man durch Vermahlen des Wirkstoffes mit feinverteilten, festen Stoffen z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrationen mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel,

Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel-, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Auch Mischungen oder Mischformulierungen mit anderen Wirkstoffen, wie z.B. Insektiziden, Akariziden, Herbiziden, Düngemitteln, Wachstumsregulatoren oder Fungiziden sind gegebenenfalls möglich.

Die Erfindung wird durch nachstehende Beispiel näher erläutert.

**Formulierungsbeispiele**

A. Ein Stäubemittel wird erhalten, indem man 10 Gewichtsteile Wirkstoff und 90 Gewichtsteile Talkum oder Inerstoff mischt und in einer Schlagmühle zerkleinert.

B. Ein in Wasser leicht dispergierbares, benetzbares Pulver wird erhalten, indem man 25 Gewichtsteile Wirkstoff, 64 Gewichtsteile kaolinhaltigen Quarz aus Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

C. Ein in Wasser leicht dispergierbares Dispersionskonzentrat wird erhalten, indem man 20 Gewichtsteile Wirkstoff mit 6 Gewichtsteilen Alkylphenolpolyglykolether (Triton x 207), 3 Gewichtsteilen Isotridecanolpolyglykolether (8 AeO) und 71 Gewichtsteilen paraffinischem Mineralöl (Siedebereich z.B. ca. 255 bis über 377 °C) mischt und in einer Reibkugelmühle auf eine Feinheit von unter 5 Mikron vermahlt.

D. Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

**Chemische Beispiele**

**Beispiel 1**

N-(1,4-Butansultam)-sulfonylisocyanat

Zu einer Suspension von 6,78 g (0.05 mol) Butansultam-(1,4) - hergestellt nach Liebigs Ann. 651 - (1962), S. 26 - in 80 ml abs. Chlorbenzol tropft man bei 0 °C eine Lösung von 7,78 g (0.055 mol) Chlorsulfonylisocyanat in 20 ml abs. Chlorbenzol. Nach Beendigung des Zutropfens steigert man die Temperatur langsam auf 125-130 °C und erhitzt ca. 6 h auf 130 °C. Man kühlt ab und entfernt das Lösungsmittel am Rotationsverdampfer. Das zurückbleibende Öl (12.0 g ≙ 100 % d.Th.) wird ohne Reinigung eingesetzt.

**Beispiel 2**

1-[N-(1,4-Butansultam)-sulfonyl]-3-(4,6-dimethyl-pyrimidin-2-yl)-harnstoff

Zu 3,08 g (0.025 mol) 2-Amino-4,6-dimethylpyrimidin in 80 ml abs. Dichlormethan tropft man bei 0 °C eine Lösung von 6,24 g (0,026 mol) N-(1,4-Butansultam)-sulfonylisocyanat in 20 ml Dichlormethan. Man läßt auf Raumtemperatur kommen und rührt 18 Stunden nach. Die Reaktionslösung wird mit 0,5 N Salzsäure und Wasser gewaschen und über Natriumsulfat getrocknet, anschließend wird das Lösungsmittel am Rotationsverdampfer entfernt. Das zurückbleibende Öl wird aus Dichlormethan/n-Heptan auskristallisiert. Man erhält 8,36 g (92 % d.Th.) 1-[N-(1,4-Butansultam)-sulfonyl]-3-(4,6-dimethyl-pyrimidin-2-yl)-harnstoff vom Schmp. 145-147 °C.

**Beispiel 3**

1-[N-(1,3-Propansultam)-sulfonyl]-3-(4,6-dimethyl-pyrimidin-2-yl)-harnstoff

2,2 ml (0,025 mol) Chlorsulfonylisocyanat werden in 100 ml abs. Acetonitril bei 40 °C vorgelegt und unter Stickstoff mit 3,88 g (0,025 mol) 2-Amino-4,6-dimethoxypyrimidin versetzt. Diese Suspension wird ca. 1 h bei 0 °C gerührt, auf -40 °C gekühlt und mit 3,07 g (0,025 mol) 1,3-Propansultam gelöst in 20 ml abs. $CH_3CN$, versetzt. Man läßt innerhalb von 4 h auf Raumtemperatur kommen und 18 h bei Raumtemperatur nach. Der Rückstand wird abgesaugt, in 50 ml abs. Acetonitril bei 0 °C suspendiert und mit 3,5 ml (0,025 mol) Triethylamin versetzt. Nach 2 h Rühren bei Raumtemperatur wird abgesaugt, der Rückstand mit Wasser gewaschen und im Vakuum getrocknet. Man erhält 9,21 g (96,6 % d.Th.) 1-[N-(1,3-Propansultam)-sulfonyl]-3-(4,6-dimethoxy-pyrimidin-2-yl)-harnstoff vom Schmp. 166-170 °C.

**Tabelle 1**

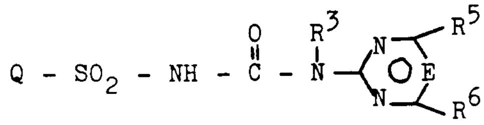

| Bsp. | Q | $R^3$ | $R^5$ | $R^6$ | E | Fp |
|---|---|---|---|---|---|---|
| 4 | 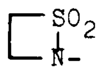 | H | $CH_3$ | $CH_3$ | CH | |
| 5 | 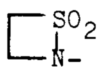 | H | $OCH_3$ | $CH_3$ | CH | |
| 6 | 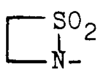 | H | $OCH_3$ | $OCH_3$ | CH | 40-44 |
| 7 | 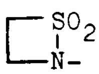 | H | $OCH_3$ | Cl | CH | |
| 8 | 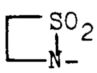 | H | $OC_2H_5$ | $OCH_3$ | CH | |
| 9 | 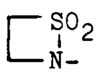 | H | $OCHF_2$ | $OCH_3$ | CH | |
| 10 | 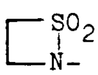 | H | $OCHF_2$ | $OCHF_2$ | CH | |
| 11 | 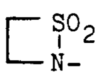 | H | $OCH_2CF_3$ | $OCH_3$ | CH | |
| 12 | 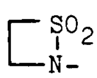 | H | $CH_3$ | Cl | CH | |
| 13 | 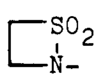 | H | $CH_3$ | $CH_3$ | N | |
| 14 | 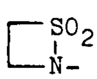 | H | $OCH_3$ | $CH_3$ | N | |
| 15 | 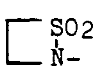 | H | OCH3 | OCH3 | N | |

**Tabelle 1** (Fortsetzung)

| Bsp. | Q | $R^3$ | $R^5$ | $R^6$ | E | Fp |
|---|---|---|---|---|---|---|
| 16 | 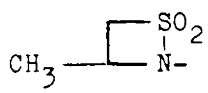 | H | $CH_3$ | $CH_3$ | CH | |
| 17 | 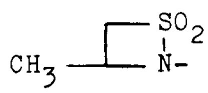 | H | $OCH_3$ | $CH_3$ | CH | |
| 18 | 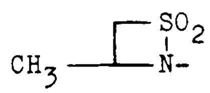 | H | $OCH_3$ | $OCH_3$ | CH | 68-71 |
| 19 | 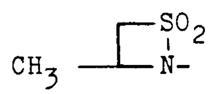 | $CH_3$ | $OCH_3$ | $OCH_3$ | CH | |
| 20 | 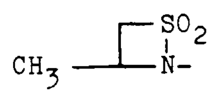 | H | $OCH_3$ | Cl | CH | |
| 21 | 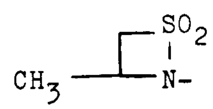 | H | $OCH_3$ | $NHCH_3$ | CH | |
| 22 | 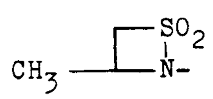 | H | $OCH_3$ | Br | CH | |
| 23 | 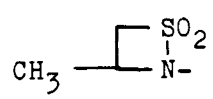 | H | $OCH_3$ | $SCH_3$ | CH | |
| 24 | 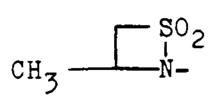 | H | $OCHF_2$ | $OCH_3$ | CH | |
| 25 | 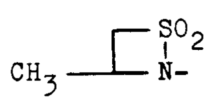 | H | $OCH_3$ | $CH_3$ | N | |
| 26 | 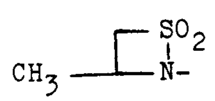 | H | $OC_2H_5$ | $NHCH_3$ | N | |
| 27 | 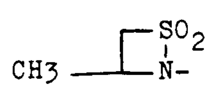 | H | $OCH_3$ | $N(CH_3)_2$ | N | |

**Tabelle 1** (Fortsetzung)

| Bsp. | Q | $R^3$ | $R^5$ | $R^6$ | E | Fp |
|---|---|---|---|---|---|---|
| 28 | 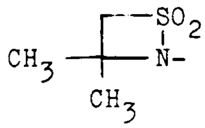 | H | $CH_3$ | $CH_3$ | CH | |
| 29 | 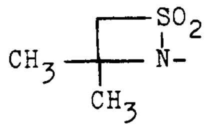 | H | $OCH_3$ | $CH_3$ | CH | |
| 30 | 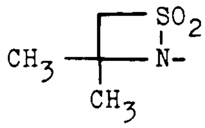 | H | $OCH_3$ | $OCH_3$ | CH | 131-133 |
| 31 | 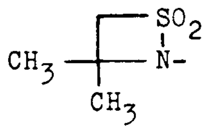 | $CH_3$ | $OCH_3$ | $CH_3$ | CH | |
| 32 | 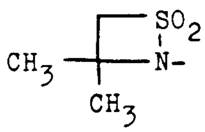 | H | $OCH_3$ | Cl | CH | |
| 33 | 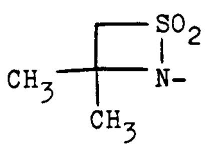 | H | $OCHF_2$ | $OCHF_2$ | CH | |
| 34 | 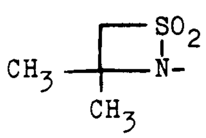 | H | $SCH_3$ | $CH_3$ | CH | |
| 35 | 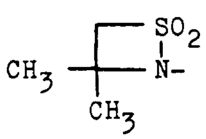 | H | $CH(OCH_3)_2$ | $OCH_3$ | CH | |
| 36 | 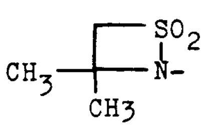 | H | $OCH_3$ | $CH_3$ | N | |

**Tabelle 1** (Fortsetzung)

| Bsp. | Q | $R^3$ | $R^5$ | $R^6$ | E | Fp |
|---|---|---|---|---|---|---|
| 37 | 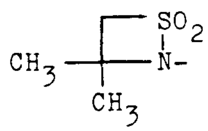 | H | $OCH_3$ | $OCH_3$ | N | |
| 38 | 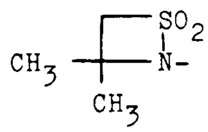 | H | $NH_2$ | $OCH_3$ | N | |
| 39 | 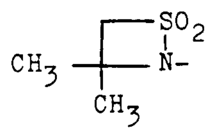 | $CH_3$ | $OCH_3$ | $CH_3$ | N | |
| 40 | 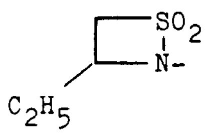 | H | $OCH_3$ | $OCH_3$ | CH | |
| 41 | 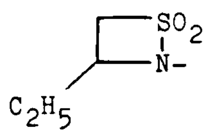 | H | $OCH_3$ | $CH_3$ | CH | |
| 42 | 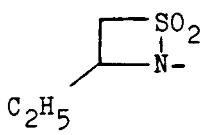 | H | $CH_3$ | $CH_3$ | CH | |
| 43 | 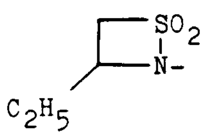 | H | $OCH_3$ | $CH_3$ | N | |
| 44 | 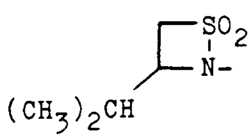 | H | $OCH_3$ | $OCH_3$ | CH | |
| 45 | 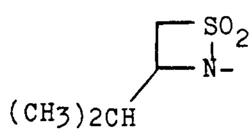 | H | $OCH_3$ | Cl | CH | |

**Tabelle 1** (Fortsetzung)

| Bsp. | Q | $R^3$ | $R^5$ | $R^6$ | E | Fp |
|---|---|---|---|---|---|---|
| 46 | 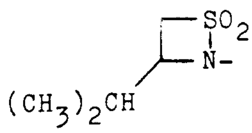 | H | $OCH_3$ | $CH_3$ | CH | |
| 47 | 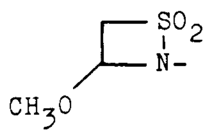 | H | $OCH_3$ | $OCH_3$ | CH | |
| 48 | 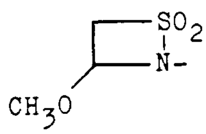 | H | $CH_3$ | $CH_3$ | CH | |
| 49 | 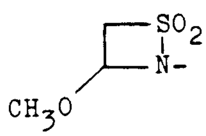 | H | $OCH_3$ | Cl | CH | |
| 50 | 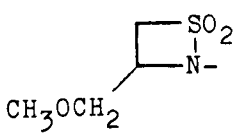 | H | $OCH_3$ | $OCH_3$ | CH | |
| 51 | 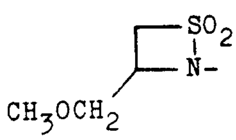 | H | $OCH_3$ | $CH_3$ | N | |
| 52 | 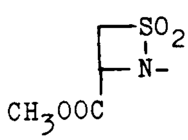 | H | $OCH_3$ | $OCH_3$ | CH | 110-111 |
| 53 | 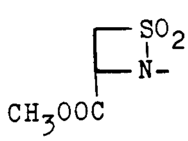 | H | $OCH_3$ | $OCH_3$ | CH | |
| 54 | 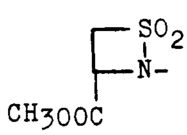 | H | $OCH_3$ | Cl | CH | |

**Tabelle 1** (Fortsetzung)

| Bsp. | Q | $R^3$ | $R^5$ | $R^6$ | E | Fp |
|---|---|---|---|---|---|---|
| 55 | 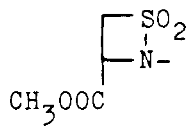 | H | $OC_2H_5$ | Cl | CH | |
| 56 | 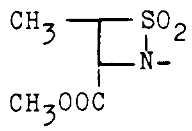 | H | $OCH_3$ | $OCH_3$ | CH | |
| 57 | 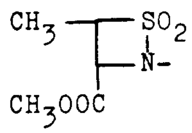 | H | $OCH_3$ | $CH_3$ | N | |
| 58 | 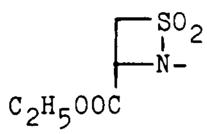 | H | $OCH_3$ | $OCH_3$ | CH | |
| 59 | 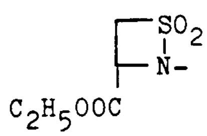 | H | $CH_3$ | $CH_3$ | CH | |
| 60 | 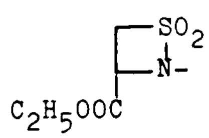 | H | $OCH_3$ | $OCH_3$ | N | |
| 61 | 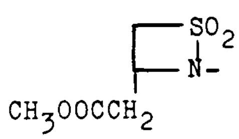 | H | $OCH_3$ | $OCH_3$ | CH | |
| 62 | 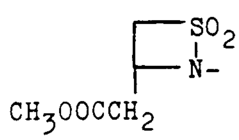 | H | $OCH_3$ | $ClCH_2$ | N | |
| 63 | 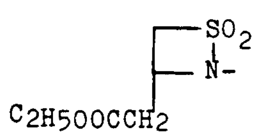 | H | $OCH_3$ | $OCH_3$ | CH | |

**Tabelle 1** (Fortsetzung)

| Bsp. | Q | $R^3$ | $R^5$ | $R^6$ | E | Fp |
|---|---|---|---|---|---|---|
| 64 | 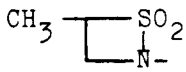 | H | $OCH_3$ | $OCH_3$ | CH | |
| 65 | 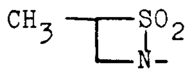 | H | $OCH_3$ | $CH_3$ | CH | |
| 66 | 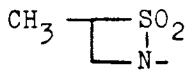 | H | $CH_3$ | $CH_3$ | CH | |
| 67 | 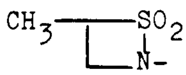 | $CH_3$ | $OCH_3$ | $CH_3$ | N | |
| 68 | 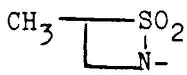 | $C_2H_5$ | $OCH_3$ | $OCH_3$ | N | |
| 69 | 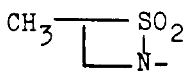 | H | $OCH_3$ | $CH_3$ | N | |
| 70 | 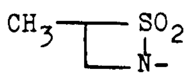 | H | $OC_2H_5$ | $NHCH_3$ | N | |
| 71 | 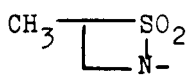 | H | $OCH_3$ | $NHCH_3$ | CH | |
| 72 | 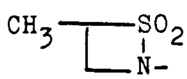 | H | Cl | $SCH_3$ | CH | |
| 73 | 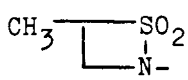 | H | Cl | $OCH_3$ | CH | |
| 74 | 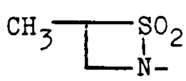 | H | $OCHF_2$ | $OCHF_2$ | CH | |
| 75 | 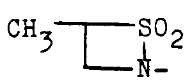 | H | 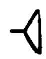 | $OCH_3$ | N | |

**Tabelle 1** (Fortsetzung)

| Bsp. | Q | $R^3$ | $R^5$ | $R^6$ | E | Fp |
|---|---|---|---|---|---|---|
| 76 | 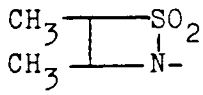 | H | $OCH_3$ | $OCH_3$ | CH | |
| 77 | 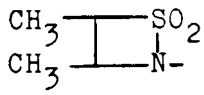 | H | $OCH_3$ | $OCH_3$ | CH | |
| 78 | 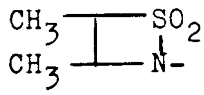 | H | $OCH_3$ | Cl | CH | |
| 79 | 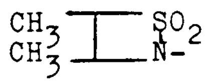 | H | $OCH_3$ | $CH_3$ | N | |
| 80 | 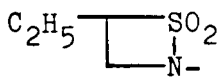 | H | $OCH_3$ | $OCH_3$ | CH | |
| 81 | 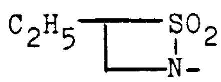 | H | $OCH_3$ | Cl | CH | |
| 82 | 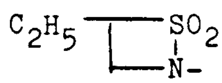 | H | $OCHCF_2$ | $OCH_3$ | CH | |
| 83 | 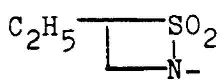 | H | $CH_3$ | $CH_3$ | CH | |
| 84 | 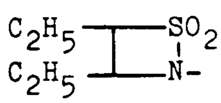 | H | $OCH_3$ | $OCH_3$ | CH | |
| 85 | 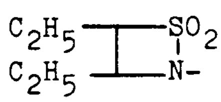 | H | $OCH_3$ | Cl | CH | |
| 86 | 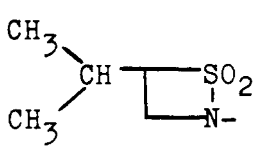 | H | $OCH_3$ | $OCH_3$ | CH | |

**Tabelle 1** (Fortsetzung)

| Bsp. | Q | $R^3$ | $R^5$ | $R^6$ | E | Fp |
|---|---|---|---|---|---|---|
| 87 | 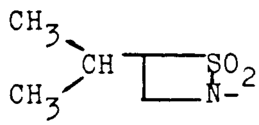 | H | $OCH_3$ | $CH_3$ | N | |
| 88 | 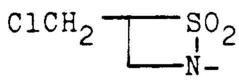 | H | $OCH_3$ | $OCH_3$ | CH | |
| 89 | 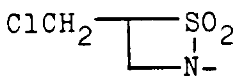 | H | $OCH_3$ | $CH_3$ | CH | |
| 90 | 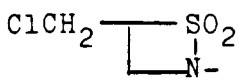 | H | $OCH_3$ | $CH_3$ | N | |
| 91 | 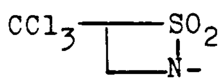 | H | $OCH_3$ | $OCH_3$ | CH | |
| 92 | 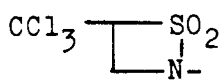 | H | $OCH_3$ | Cl | CH | |
| 93 | 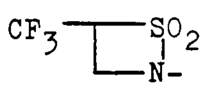 | H | $OCH_3$ | $OCH_3$ | CH | |
| 94 | 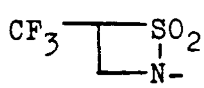 | H | $CH_3$ | $CH_3$ | CH | |
| 95 | 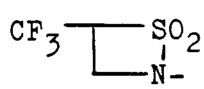 | H | $OCH_3$ | $CH_3$ | N | |
| 96 | 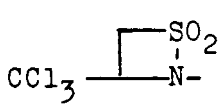 | H | $OCH_3$ | $OCH_3$ | CH | |
| 97 | 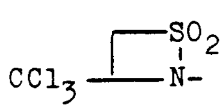 | H | $CH_3$ | $CH_3$ | CH | |

**Tabelle 1** (Fortsetzung)

| Bsp. | Q | $R^3$ | $R^5$ | $R^6$ | E | Fp |
|---|---|---|---|---|---|---|
| 98 | 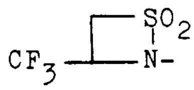 | H | $OCH_3$ | $OCH_3$ | CH | |
| 99 | 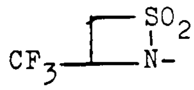 | H | $OCH_3$ | $CH_3$ | CH | |
| 100 | 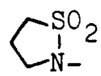 | H | $OCH_3$ | $CH_3$ | CH | |
| 101 | 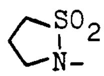 | H | $CH_3$ | $CH_3$ | CH | 161-163 |
| 102 | 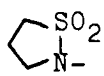 | H | $OCH_3$ | Cl | CH | 148-149 |
| 103 | 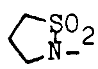 | H | $CH_3$ | Cl | CH | |
| 104 | 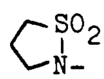 | H | $OCHF_2$ | Cl | CH | |
| 105 | 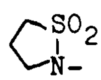 | H | $OCHF_2$ | $OCH_3$ | CH | |
| 106 | 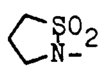 | H | $OCHF_2$ | $OCHF_2$ | CH | |
| 107 | 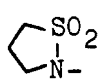 | H | $OC_2H_5$ | Cl | CH | |
| 108 | 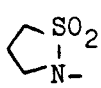 | H | $OC_2H_5$ | $CH_3$ | CH | |
| 109 | 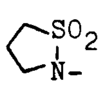 | H | $OC_2H_5$ | $OCH_3$ | CH | |

**Tabelle 1** (Fortsetzung)

| Bsp. | Q | $R^3$ | $R^5$ | $R^6$ | E | Fp |
|---|---|---|---|---|---|---|
| 110 | 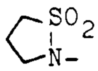 | H | $OCH_3$ | $SCH_3$ | CH | |
| 111 | 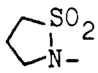 | H | $OCH_3$ | 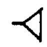 | CH | |
| 112 | 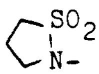 | H | $OCH_3$ | $NHCH_3$ | CH | |
| 113 | 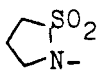 | H | $OCH_3$ | $H(CH_3)_2$ | CH | |
| 114 | 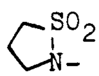 | H | $OCH_3$ | $NH_2$ | CH | |
| 115 | 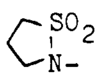 | $CH_3$ | $OCH_3$ | $OCH_3$ | CH | |
| 116 | 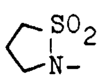 | $CH_3$ | $OCH_3$ | $CH_3$ | CH | |
| 117 | 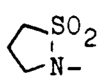 | $CH_2-CH=CH_2$ | $OCH_3$ | $OCH_3$ | CH | |
| 118 | 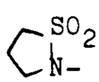 | $OCH_3$ | $OCH_3$ | $OCH_3$ | CH | |
| 119 | 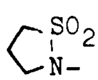 | H | $OCH_3$ | $OCH_3$ | N | |
| 120 | 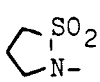 | H | $OCH_3$ | $CH_3$ | N | |
| 121 | 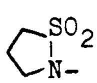 | H | $CH_3$ | $CH_3$ | N | |

**Tabelle 1** (Fortsetzung)

| Bsp. | Q | $R^3$ | $R^5$ | $R^6$ | E | Fp |
|---|---|---|---|---|---|---|
| 122 | 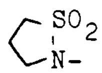 | H | $OCH_3$ | $NHCH_3$ | N | |
| 123 | 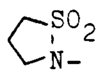 | H | $OCH_3$ | $N(CH_3)_2$ | N | |
| 124 | 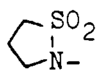 | H | $OC_2H_5$ | $NHCH_3$ | N | |
| 125 | 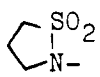 | H | $OCH_3$ | $OCH_2CF_3$ | N | |
| 126 | 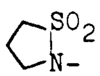 | H | $OCH_3$ | $OCHF_2$ | N | |
| 127 | 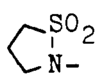 | H | $OCH_3$ | $CH_2Cl$ | N | |
| 128 | 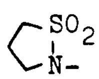 | H | $OCH_3$ | 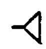 | N | |
| 129 | 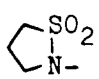 | H | $OCH_3$ | $CH(OCH_3)_2$ | N | |
| 130 | 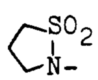 | H | $SCH_3$ | $CH_3$ | N | |
| 131 | 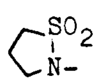 | H | $SCH_3$ | $C_2H_5$ | N | |
| 132 | 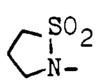 | H | $SCH_3$ | $OCH_3$ | N | |
| 133 | 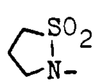 | H | $SCH_3$ | $OC_2H_5$ | N | |

**Tabelle 1** (Fortsetzung)

| Bsp. | Q | $R^3$ | $R^5$ | $R^6$ | E | Fp |
|---|---|---|---|---|---|---|
| 134 | 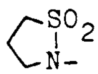 | H | $CH_2COOCH_3$ | $OCH_3$ | N | |
| 135 | 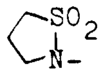 | H | $CH_2COOCH_3$ | $OC_2H_5$ | N | |
| 136 | 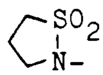 | $CH_3$ | $OCH_3$ | $OCH_3$ | N | |
| 137 | 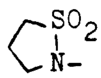 | $CH_3$ | $OCH_3$ | $CH_3$ | N | |
| 138 | 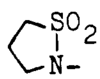 | $C_2H_5$ | $OCH_3$ | $OCH_3$ | N | |
| 139 | 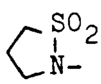 | $C_2H_5$ | $OCH_3$ | $CH_3$ | N | |
| 140 | 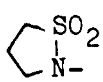 | H | $CF_3$ | $CH_3$ | CH | |
| 141 | 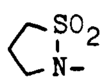 | H | $CF_3$ | $OCH_3$ | CH | |
| 142 | 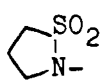 | H | $CF_3$ | $OCH_3$ | N | |
| 143 | 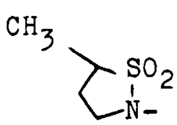 | H | $OCH_3$ | $OCH_3$ | CH | 120-124 |
| 144 | 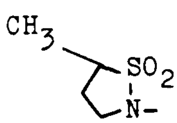 | H | $OCH_3$ | $CH_3$ | CH | |

**Tabelle 1** (Fortsetzung)

| Bsp. | Q | $R^3$ | $R^5$ | $R^6$ | E | Fp |
|---|---|---|---|---|---|---|
| 145 | 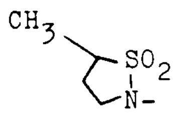 | H | $CH_3$ | $CH_3$ | CH | |
| 146 | 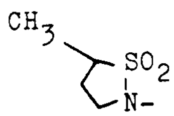 | H | $OCH_3$ | Cl | CH | |
| 147 | 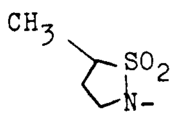 | H | $OCH_3$ | $NHCH_3$ | CH | |
| 148 | 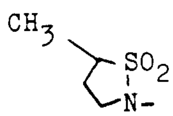 | H | $OCHF_2$ | $OCH_3$ | CH | |
| 149 | 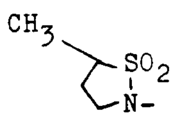 | H | $OCHF_2$ | $OCHF_2$ | CH | |
| 150 | 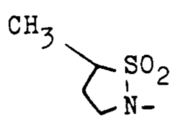 | H | $OCH_3$ | $OCH_3$ | N | |
| 151 | 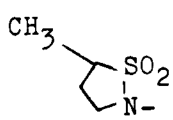 | H | $OCH_3$ | $CH_3$ | N | |
| 152 | 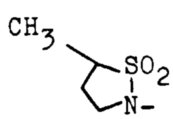 | $CH_3$ | $OCH_3$ | $CH_3$ | N | |
| 153 | 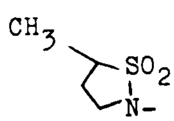 | H | $OCH_3$ | $NHCH_3$ | N | |

**Tabelle 1** (Fortsetzung)

| Bsp. | Q | $R^3$ | $R^5$ | $R^6$ | E | Fp |
|---|---|---|---|---|---|---|
| 154 | 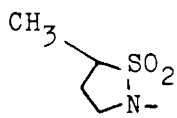 | H | $OCH_3$ | $NHC_2H_5$ | N | |
| 155 | 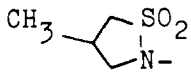 | H | $OCH_3$ | $OCH_3$ | CH | |
| 156 | 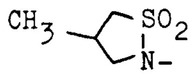 | H | $OCH_3$ | $CH_3$ | CH | |
| 157 | 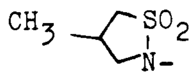 | H | $CH_3$ | $CH_3$ | CH | |
| 158 | 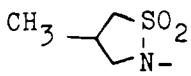 | H | $OCH_3$ | Cl | CH | |
| 159 | 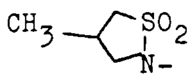 | H | $CH_3$ | Cl | CH | |
| 160 | 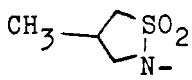 | H | $OCHF_2$ | $OCHF_2$ | CH | |
| 161 | 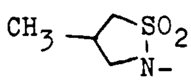 | H | $OCH_3$ | $CH_3$ | N | |
| 162 | 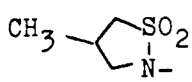 | H | $OCH_3$ | $OCH_3$ | N | |
| 163 | 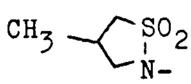 | $CH_3$ | $OCH_3$ | $CH_3$ | N | |
| 164 | 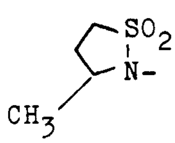 | H | $OCH_3$ | $OCH_3$ | CH | |

**Tabelle 1** (Fortsetzung)

| Bsp. | Q | $R^3$ | $R^5$ | $R^6$ | E | Fp |
|---|---|---|---|---|---|---|
| 165 | 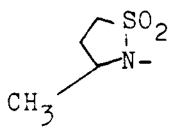 | H | $OCH_3$ | $CH_3$ | CH | |
| 166 | 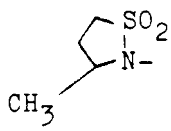 | H | $CH_3$ | $CH_3$ | CH | |
| 167 | 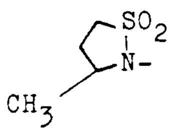 | H | $OCH_3$ | $CH_3$ | N | |
| 168 | 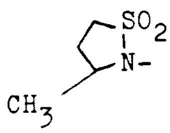 | H | $OCH_3$ | $OCH_3$ | N | |
| 169 | 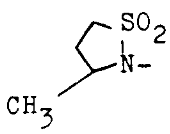 | H | $OCH_3$ | Cl | CH | |
| 170 | 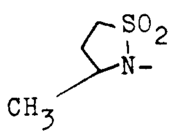 | H | $OCH_3$ | $OCH_2F_5$ | CH | |
| 171 | 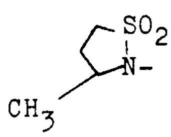 | H | $OCH_3$ | $OCH_2F_3$ | N | |
| 172 | 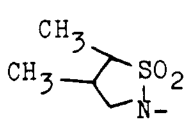 | H | $OCH_3$ | $OCH_3$ | CH | |
| 173 | 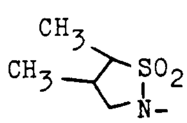 | H | $OCH_3$ | $CH_3$ | CH | |

**Tabelle 1** (Fortsetzung)

| Bsp. | Q | $R^3$ | $R^5$ | $R^6$ | E | Fp |
|---|---|---|---|---|---|---|
| 174 | 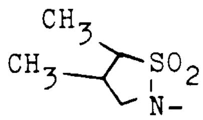 | H | $OCH_3$ | $CH_3$ | N | |
| 175 | 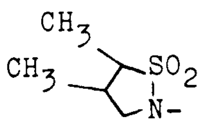 | $CH_3$ | $OCH_3$ | $CH_3$ | CH | |
| 176 | 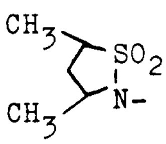 | H | $OCH_3$ | $OCH_3$ | CH | |
| 177 | 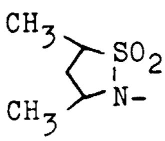 | H | $CH_3$ | $CH_3$ | CH | |
| 178 | 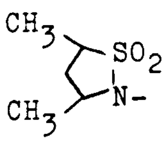 | H | $OCH_3$ | $OCH_3$ | N | |
| 179 | 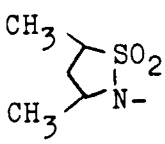 | H | $OCH_3$ | $CH_3$ | N | |
| 180 | 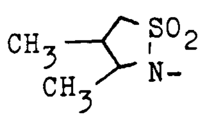 | H | $OCH_3$ | $OCH_3$ | CH | |
| 181 | 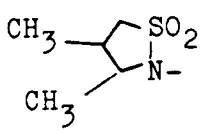 | H | $OCH_3$ | $CH_3$ | CH | |
| 182 | 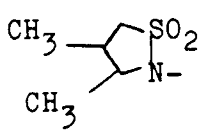 | H | $OCH_3$ | Cl | CH | |

Tabelle 1 (Fortsetzung)

| Bsp. | Q | $R^3$ | $R^5$ | $R^6$ | E | Fp |
|---|---|---|---|---|---|---|
| 183 | 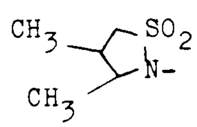 | H | $OCH_3$ | 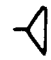 | N | |
| 184 | 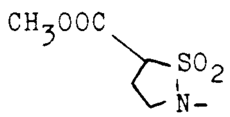 | H | $OCH_3$ | $OCH_3$ | CH | |
| 185 | 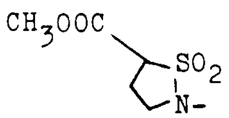 | H | $OCH_3$ | $OCH_3$ | N | |
| 186 | 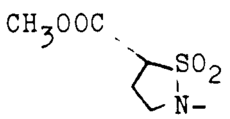 | H | $OCH_3$ | Cl | CH | |
| 187 | 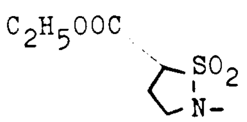 | H | $OCH_3$ | $OCH_3$ | CH | |
| 188 | 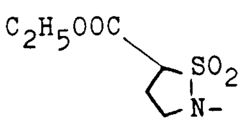 | H | $CH_3$ | $CH_3$ | CH | |
| 189 | 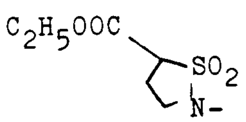 | H | $CH_3$ | 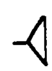 | CH | |
| 190 | 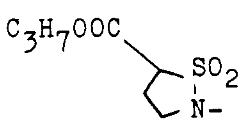 | H | $OCH_3$ | $OCH_3$ | CH | |
| 191 | 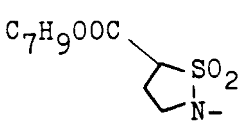 | H | $OCH_3$ | $OCH_3$ | CH | |

**Tabelle 1** (Fortsetzung)

| Bsp. | Q | $R^3$ | $R^5$ | $R^6$ | E | Fp |
|---|---|---|---|---|---|---|
| 192 | 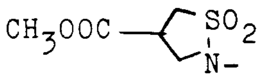 | H | $OCH_3$ | $OCH_3$ | CH | |
| 193 | 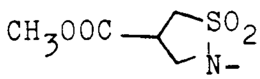 | H | $CH_3$ | $CH_3$ | CH | |
| 194 | 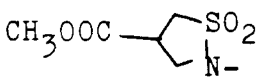 | H | $OCH_3$ | Cl | CH | |
| 195 | 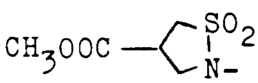 | H | $OCH_3$ | $CH_3$ | N | |
| 196 | 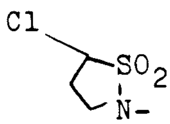 | H | $OCH_3$ | $OCH_3$ | CH | |
| 197 | 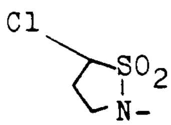 | H | $OCH_3$ | $CH_3$ | CH | |
| 198 | 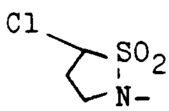 | H | $OCH_3$ | Cl | CH | |
| 199 | 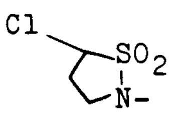 | H | $OCH_3$ | $CH_3$ | N | |
| 200 | 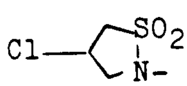 | H | $OCH_3$ | $OCH_3$ | CH | |
| 201 | 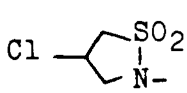 | H | $OCH_3$ | $CH_3$ | N | |

**Tabelle 1** (Fortsetzung)

| Bsp. | Q | $R^3$ | $R^5$ | $R^6$ | E | Fp |
|---|---|---|---|---|---|---|
| 202 | 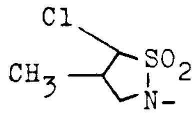 | H | $OCH_3$ | $OCH_3$ | CH | |
| 203 | 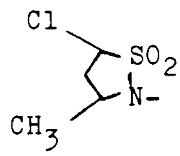 | H | $OCH_3$ | $OCH_3$ | CH | |
| 204 | 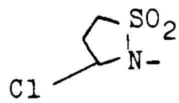 | H | $OCH_3$ | $OCH_3$ | CH | |
| 205 | 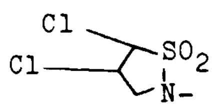 | H | $OCH_3$ | $OCH_3$ | CH | |
| 206 | 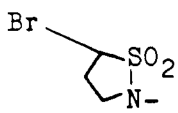 | H | $OCH_3$ | $OCH_3$ | CH | |
| 207 | 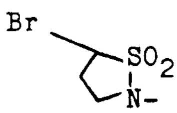 | H | $OCH_3$ | $CH_3$ | N | |
| 208 | 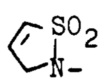 | H | $OCH_3$ | $OCH_3$ | CH | |
| 209 | 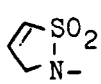 | H | $OCH_3$ | Cl | CH | |
| 210 | 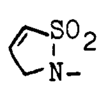 | H | $OCH_3$ | $CH_3$ | N | |
| 211 | 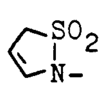 | H | $OCH_3$ | $OCH_3$ | CH | |

**Tabelle 1** (Fortsetzung)

| Bsp. | Q | $R^3$ | $R^5$ | $R^6$ | E | Fp |
|---|---|---|---|---|---|---|
| 212 | 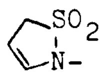 | H | $OCH_3$ | Cl | CH | |
| 213 | 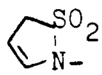 | H | $OCH_3$ | $NHCH_3$ | N | |
| 214 | 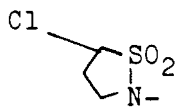 | H | $OCH_3$ | $OCH_3$ | CH | |
| 215 | 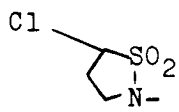 | H | $OCH_3$ | $CH_3$ | N | |
| 216 | 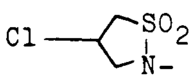 | H | $OCH_3$ | $OCH_3$ | CH | |
| 217 | 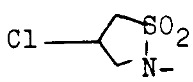 | H | $OCH_3$ | $CH_3$ | N | |
| 218 | 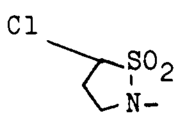 | H | $OCH_3$ | $OCH_3$ | CH | |
| 219 | 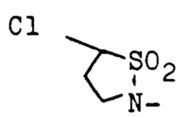 | H | $OCH_3$ | $CH_3$ | N | |
| 220 | 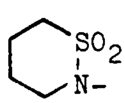 | H | $OCH_3$ | $CH_3$ | CH | 172-173 |
| 221 | 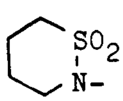 | H | $OCH_3$ | $CH_3$ | CH | 162-164 |

**Tabelle 1** (Fortsetzung)

| Bsp. | Q | $R^3$ | $R^5$ | $R^6$ | E | Fp |
|---|---|---|---|---|---|---|
| 222 | 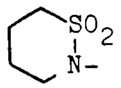 | H | $OCH_3$ | Cl | CH | 148-149 |
| 223 | 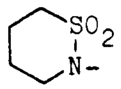 | H | $CH_3$ | Cl | CH | |
| 224 | 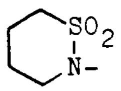 | H | $CH_3$ | Br | CH | |
| 225 | 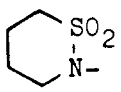 | H | $OCH_3$ | Br | CH | |
| 226 | 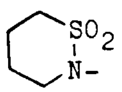 | H | $OCHF_2$ | $OCHF_2$ | CH | |
| 227 | 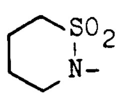 | H | $OCHF_2$ | $OCH_3$ | CH | |
| 228 | 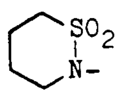 | H | $OCH_3$ | $OCH_2CF_3$ | CH | |
| 229 | 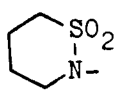 | H | $OC_2H_5$ | $OCHCF_3$ | CH | |
| 230 | 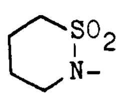 | H | $OC_2H_5$ | $OC_2H_5$ | CH | |
| 231 | 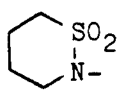 | H | $SCH_3$ | $CH_3$ | CH | |
| 232 | 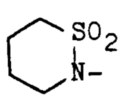 | H | $OCH_3$ | $NHCH_3$ | CH | |
| 233 | 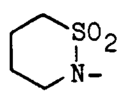 | H | $CH_3$ | $N(CH_3)_2$ | CH | |

**Tabelle 1** (Fortsetzung)

| Bsp. | Q | $R^3$ | $R^5$ | $R^6$ | E | Fp |
|---|---|---|---|---|---|---|
| 234 | 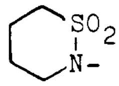 | H | $OCH_3$ | 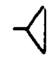 | CH | |
| 235 | 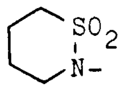 | H | $CH_3$ | 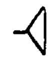 | CH | |
| 236 | 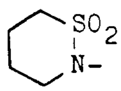 | H | $OCHF_2$ | $OCHF_2$ | CH | |
| 237 | 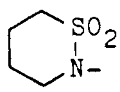 | $CH_3$ | $OCH_3$ | $OCH_3$ | CH | |
| 238 | 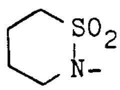 | $CH_3$ | $CH_3$ | $CH_3$ | CH | |
| 239 | 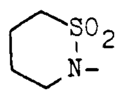 | H | $OCH_3$ | $OCH_3$ | N | |
| 240 | 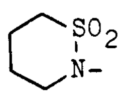 | H | $OCH_3$ | $CH_3$ | N | |
| 241 | 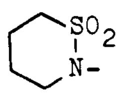 | H | $OCH_3$ | $SCH_3$ | N | |
| 242 | 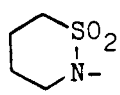 | H | $CH_3$ | $CH_3$ | N | |
| 243 | 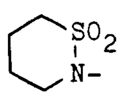 | H | $OC_2H_5$ | $OCH_3$ | N | |
| 244 | 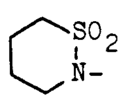 | H | $OCH_3$ | $NHCH_3$ | N | |
| 245 | 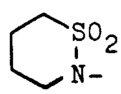 | H | $OC_2H_5$ | $NHCH_3$ | N | |

Tabelle 1 (Fortsetzung)

| Bsp. | Q | $R^3$ | $R^5$ | $R^6$ | E | Fp |
|---|---|---|---|---|---|---|
| 246 | 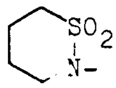 | H | $OCH_3$ | $NHC_2H_5$ | N | |
| 247 | 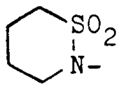 | H | $OC_2H_5$ | $NHC_2H_5$ | N | |
| 248 | 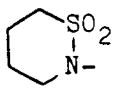 | H | $OCH_3$ | $N(CH_3)_2$ | N | |
| 249 | 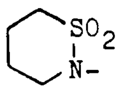 | H | $OC_2H_5$ | $N(CH_3)_2$ | N | |
| 250 | 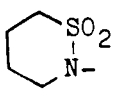 | H | $OCH_3$ | - | N | |
| 251 | 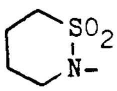 | H | $OCH_3$ | $OCH_2CF_3$ | N | |
| 252 | 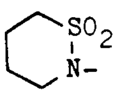 | H | $CH_3$ | $OCH_2CF_3$ | N | |
| 253 | 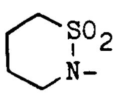 | $CH_3$ | $OCH_3$ | $CH_3$ | N | |
| 254 | 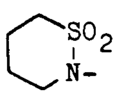 | $CH_3$ | $OCH_3$ | $OCH_3$ | N | |
| 255 | 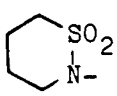 | $CH_3$ | $OCH_3$ | $NHCH_3$ | N | |
| 256 | 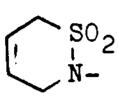 | H | $OCH_3$ | $OCH_3$ | CH | 48-50 |
| 257 | 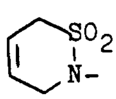 | H | $OCH_3$ | $CH_3$ | CH | |

**Tabelle 1** (Fortsetzung)

| Bsp. | Q | $R^3$ | $R^5$ | $R^6$ | E | Fp |
|---|---|---|---|---|---|---|
| 258 | 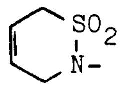 | H | $CH_3$ | $CH_3$ | CH | |
| 259 | 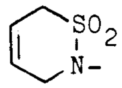 | H | $OCH_3$ | Cl | CH | |
| 260 | 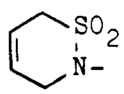 | H | $OCH_3$ | Br | CH | |
| 261 | 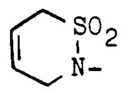 | H | $OC_2H_5$ | $OCH_3$ | CH | |
| 262 | 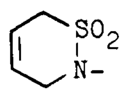 | H | $OC_2H_5$ | $OC_2H_5$ | CH | |
| 263 | 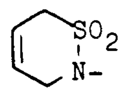 | H | $OCH_3$ | $NHCH_3$ | CH | |
| 264 | 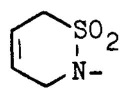 | H | $OCH_3$ | $CH_3$ | N | |
| 265 | 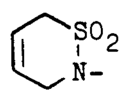 | H | $OCH_3$ | $NHCH_3$ | N | |
| 266 | 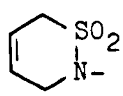 | $CH_3$ | $OCH_3$ | $CH_3$ | N | |
| 267 | 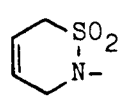 | H | $OCH_3$ | $OCH_2CF_3$ | CH | |
| 268 | 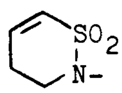 | H | $OCH_3$ | $OCH_3$ | CH | |
| 269 | 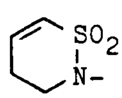 | H | $OCH_3$ | $CH_3$ | CH | |

**Tabelle 1** (Fortsetzung)

| Bsp. | Q | $R^3$ | $R^5$ | $R^6$ | E | Fp |
|---|---|---|---|---|---|---|
| 270 | 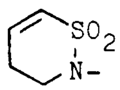 | H | $CH_3$ | $CH_3$ | CH | |
| 271 | 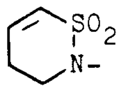 | H | $OCH_3$ | Cl | CH | |
| 272 | 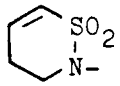 | H | $CH_3$ | Cl | CH | |
| 273 | 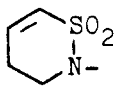 | H | $OCH_3$ | $OCH_2CF_3$ | CH | |
| 274 | 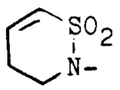 | H | $OCHF_2$ | $OCHF_2$ | CH | |
| 275 | 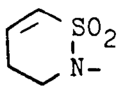 | H | $OCH_3$ | $CH_3$ | N | |
| 276 | 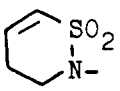 | $CH_3$ | $OCH_3$ | $CH_3$ | N | |
| 277 | 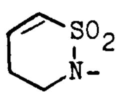 | H | $OH_3$ | 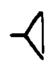 | N | |
| 278 | 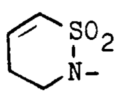 | H | $OC_2H_5$ | $NHCH_3$ | N | |
| 279 | 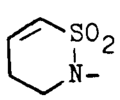 | H | $SCH_3$ | $CH_3$ | N | |
| 280 | 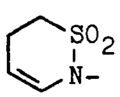 | H | $OCH_3$ | $OCH_3$ | CH | |
| 281 | 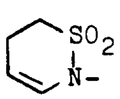 | H | $OCH_3$ | $CH_3$ | CH | |

Tabelle 1 (Fortsetzung)

| Bsp. | Q | $R^3$ | $R^5$ | $R^6$ | E | Fp |
|---|---|---|---|---|---|---|
| 282 | 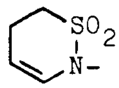 | H | $CH_3$ | $CH_3$ | CH | |
| 283 | 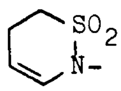 | H | $OCH_3$ | Cl | CH | |
| 284 | 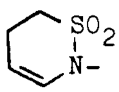 | H | $OCHF_2$ | $OCHF_2$ | CH | |
| 285 | 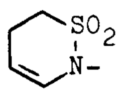 | H | $SCH_3$ | $CH_3$ | CH | |
| 286 | 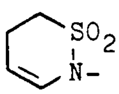 | H | $OC_2H_5$ | $NHCH_3$ | CH | |
| 287 | 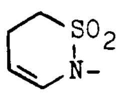 | H | $OCH_3$ | $CH_3$ | N | |
| 288 | 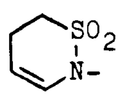 | $CH_3$ | $OCH_3$ | $CH_3$ | N | |
| 289 | 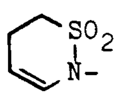 | H | $OCH_3$ | $OCH_3$ | N | |
| 290 | 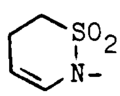 | H | $OCH_3$ | $OCH_2CF_3$ | N | |
| 291 | 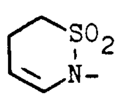 | H | $OCH_3$ | 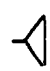 | N | |
| 292 | 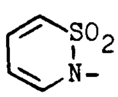 | H | $OCH_3$ | $OCH_3$ | CH | |
| 293 | 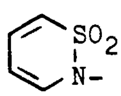 | H | $OCH_3$ | $CH_3$ | CH | |

**Tabelle 1** (Fortsetzung)

| Bsp. | Q | $R^3$ | $R^5$ | $R^6$ | E | Fp |
|---|---|---|---|---|---|---|
| 294 | 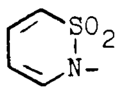 | H | $CH_3$ | $CH_3$ | CH | |
| 295 | 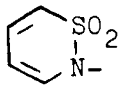 | H | $OCH_3$ | Cl | CH | |
| 296 | 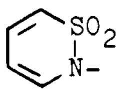 | H | $OCHF_2$ | $OCHF_2$ | CH | |
| 297 | 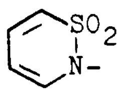 | H | $OCHF_2$ | $OCH_3$ | CH | |
| 298 | 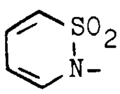 | H | $OCHF_2$ | $CH_3$ | CH | |
| 299 | 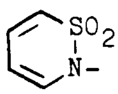 | H | $OCH_3$ | $CH_3$ | N | |
| 300 | 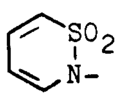 | $CH_3$ | $OCH_3$ | $CH_3$ | N | |
| 301 | 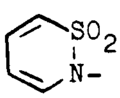 | H | $OCH_3$ | $OCH_3$ | N | |
| 302 | 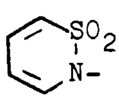 | H | $CH_3$ | $CH_3$ | N | |
| 303 | 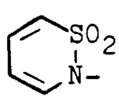 | N | H | $CH_3$ | CH | |
| 304 | 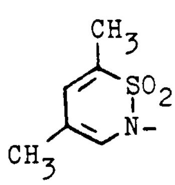 | H | $OCH_3$ | $OCH_3$ | CH | |

**Tabelle 1** (Fortsetzung)

| Bsp. | Q | $R^3$ | $R^5$ | $R^6$ | E | Fp |
|---|---|---|---|---|---|---|
| 304 | 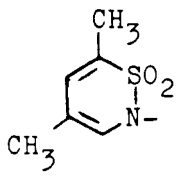 | H | $OCH_3$ | $OCH_3$ | CH | |
| 305 | 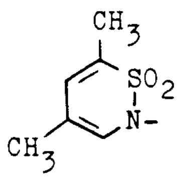 | H | $OCH_3$ | $CH_3$ | N | |
| 306 | 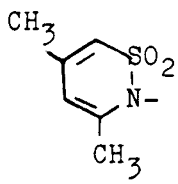 | H | $OCH_3$ | $OCH_3$ | CH | 112-114 |
| 307 | 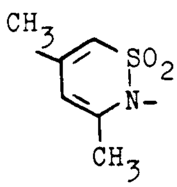 | H | $OCH_3$ | $CH_3$ | CH | |
| 308 | 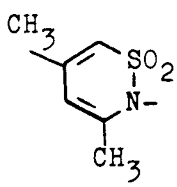 | H | $CH_3$ | $CH_3$ | CH | |
| 309 | 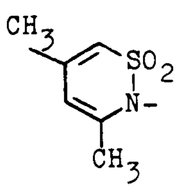 | H | $OCH_3$ | Cl | CH | |
| 310 | 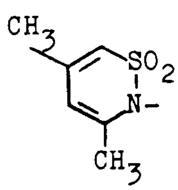 | H | $OCHF_2$ | $OCHF_2$ | CH | |

**Tabelle 1** (Fortsetzung)

| Bsp. | Q | $R^3$ | $R^5$ | $R^6$ | E | Fp |
|---|---|---|---|---|---|---|
| 311 | 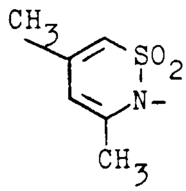 | H | $OCH_3$ | $NHCH_3$ | CH | |
| 312 | 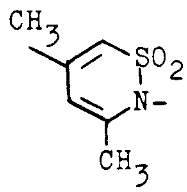 | H | $OCH_3$ | $CH_3$ | N | |
| 313 | 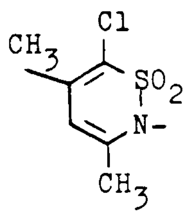 | $CH_3$ | $OCH_3$ | $CH_3$ | N | |
| 314 | 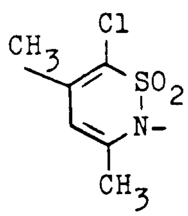 | H | $OCH_3$ | $OCH_3$ | N | |
| 315 | 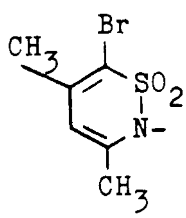 | H | $CH_3$ | $CH_3$ | N | |
| 316 | 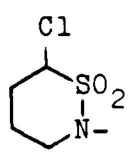 | H | $OCH_3$ | $OCH_3$ | CH | |
| 317 | 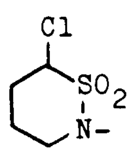 | H | $OCH_3$ | CL | CH | |

**Tabelle 1** (Fortsetzung)

| Bsp. | Q | $R^3$ | $R^5$ | $R^6$ | E | Fp |
|---|---|---|---|---|---|---|
| 318 | 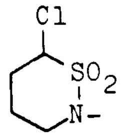 | H | $OCH_3$ | $CH_3$ | CH | |
| 319 | 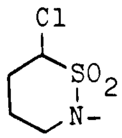 | H | $CH_3$ | $CH_3$ | CH | |
| 320 | 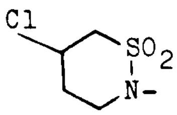 | H | $OCH_3$ | $OCH_3$ | CH | |
| 321 | 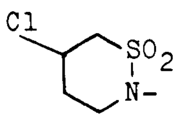 | H | $CH_3$ | $CH_3$ | CH | |
| 322 | 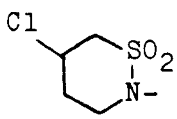 | H | $OCH_3$ | $NHCH_3$ | CH | |
| 323 | 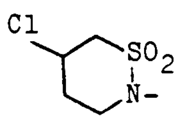 | H | $CH_3$ | H | CH | |
| 324 | 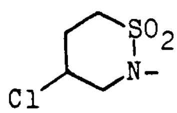 | H | $OCH_3$ | $OCH_3$ | CH | |
| 325 | 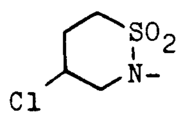 | $CH_3$ | $OCH_3$ | $CH_3$ | N | |
| 326 | 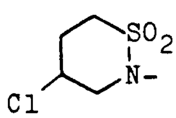 | H | $OCH_3$ | $OCH_3$ | N | |

Tabelle 1 (Fortsetzung)

| Bsp. | Q | $R^3$ | $R^5$ | $R^6$ | E | Fp |
|---|---|---|---|---|---|---|
| 327 | 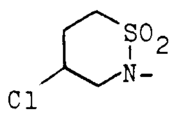 | H | $OCH_3$ | - | N | |
| 328 | 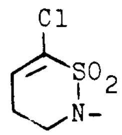 | H | $OCH_3$ | $CH_3$ | CH | |
| 329 | 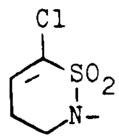 | H | $OCH_3$ | $CH_3$ | CH | |
| 330 | 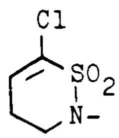 | H | $OCH_3$ | H | CH | |
| 331 | 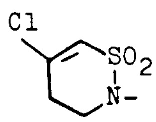 | H | $OCH_3$ | $OCH_3$ | CH | |
| 332 | 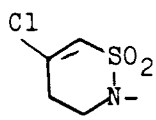 | H | $OCH_3$ | $CH_3$ | N | |
| 333 | 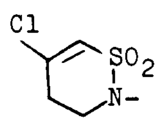 | H | $OCH_3$ | H | N | |
| 334 | 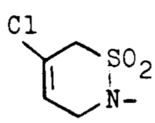 | H | $OCH_3$ | $OCH_3$ | CH | |
| 335 | 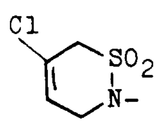 | H | $OCHF_2$ | $OCHF_2$ | CH | |

**Tabelle 1** (Fortsetzung)

| Bsp. | Q | $R^3$ | $R^5$ | $R^6$ | E | Fp |
|---|---|---|---|---|---|---|
| 336 | 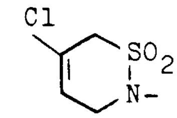 | H | $CH_3$ | $CH_3$ | CH | |
| 337 | 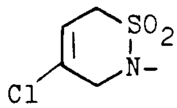 | H | $OCH_3$ | $OCH_3$ | CH | |
| 338 | 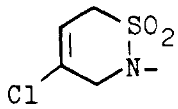 | H | $SCH_3$ | $CH_3$ | CH | |
| 339 | 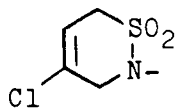 | H | $OCH_3$ | $CH_3$ | N | |
| 340 | 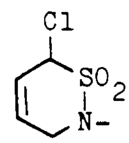 | H | $OCH_3$ | $OCH_3$ | CH | |
| 341 | 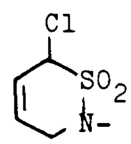 | H | $CH_3$ | $CH_3$ | CH | |
| 342 | 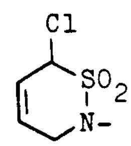 | H | $OCH_3$ | $CH_3$ | N | |
| 343 | 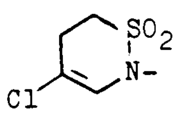 | H | $OCH_3$ | $OCH_3$ | CH | |
| 344 | 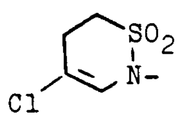 | H | $CH_3$ | $CH_3$ | CH | |

**Tabelle 1** (Fortsetzung)

| Bsp. | Q | $R^3$ | $R^5$ | $R^6$ | E | Fp |
|---|---|---|---|---|---|---|
| 345 | 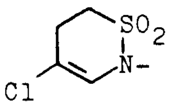 | H | $OCH_3$ | $CH_3$ | N | |
| 346 | 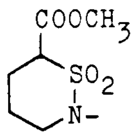 | H | $OCH_3$ | $OCH_3$ | CH | |
| 347 | 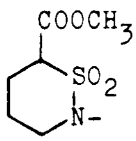 | H | $CH_3$ | $CH_3$ | CH | |
| 348 | 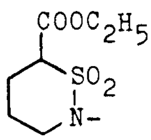 | H | $OCH_3$ | $OCH_3$ | CH | |
| 349 | 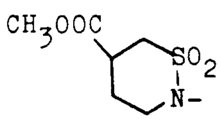 | H | $OCH_3$ | $OCH_3$ | CH | |
| 350 | 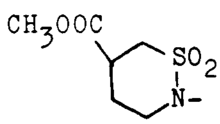 | H | $CH_3$ | $CH_3$ | CH | |
| 351 | 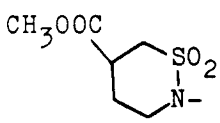 | H | $OCH_3$ | $CH_3$ | N | |
| 352 | 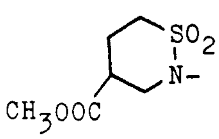 | H | $OCH_3$ | $OCH_3$ | CH | |
| 353 | 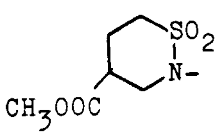 | H | $OCH_3$ | Cl | CH | |

**Tabelle 1** (Fortsetzung)

| Bsp. | Q | $R^3$ | $R^5$ | $R^6$ | E | Fp |
|---|---|---|---|---|---|---|
| 354 | 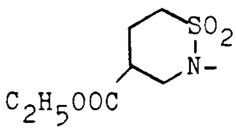 | H | $OCH_3$ | $OCH_3$ | CH | |
| 355 | 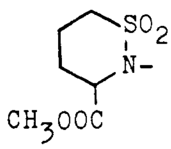 | H | $OCH_3$ | $OCH_3$ | CH | |
| 356 | 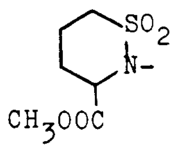 | H | $OCH_3$ | $CH_3$ | N | |
| 357 | 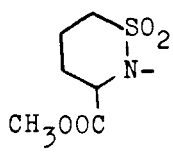 | $CH_3$ | $OCH_3$ | $CH_3$ | N | |
| 358 | 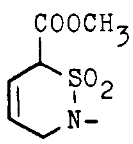 | H | $OCH_3$ | $OCH_3$ | CH | |
| 359 | 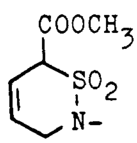 | H | $OCH_3$ | $CH_3$ | N | |
| 360 | 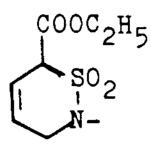 | H | $OCH_3$ | $OCH_3$ | CH | |
| 361 | 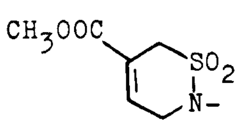 | H | $OCH_3$ | $OCH_3$ | CH | |
| 362 | 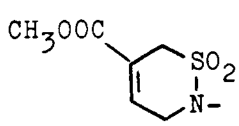 | H | $OCH_3$ | $CH_3$ | N | |

Tabelle 1 (Fortsetzung)

| Bsp. | Q | $R^3$ | $R^5$ | $R^6$ | E | Fp |
|---|---|---|---|---|---|---|
| 363 | 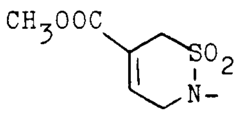 | $CH_3$ | $OCH_3$ | $OCH_3$ | CH | |
| 364 | 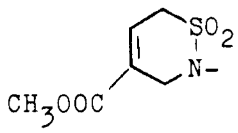 | H | $OCH_3$ | $OCH_3$ | CH | |
| 365 | 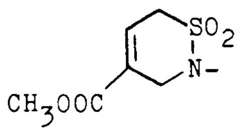 | H | $OCH_3$ | Cl | CH | |
| 366 | 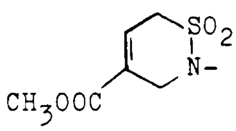 | H | $OCH_3$ | $CH_3$ | N | |
| 367 | 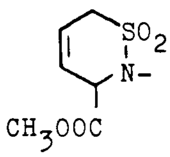 | H | $OCH_3$ | $OCH_3$ | CH | |
| 368 | 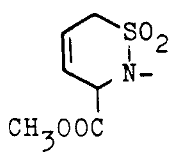 | H | $OCH_3$ | $CH_3$ | N | |
| 369 | 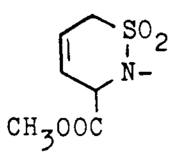 | H | $OCH_3$ | Cl | CH | |
| 370 | 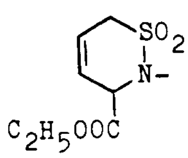 | H | $OCH_3$ | $OCH_3$ | CH | |
| 371 | 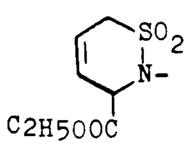 | H | $OCH_3$ | $CH_3$ | N | |

Tabelle 1 (Fortsetzung)

| Bsp. | Q | $R^3$ | $R^5$ | $R^6$ | E | Fp |
|---|---|---|---|---|---|---|
| 372 | 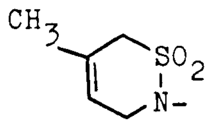 | H | $OCH_3$ | $OCH_3$ | CH | |
| 373 | 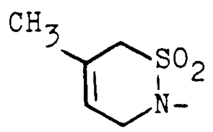 | H | $OCH_3$ | $CH_3$ | N | |
| 374 | 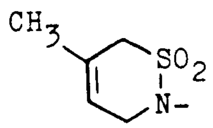 | $CH_3$ | $OCH_3$ | $CH_3$ | N | |
| 375 | 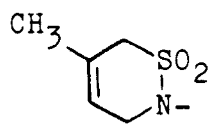 | H | $OCH_3$ | 01 | CH | |
| 376 | 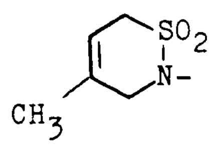 | H | $OCH_3$ | $OCH_3$ | CH | |
| 377 | 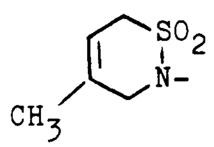 | H | $OCH_3$ | Cl | CH | |
| 378 | 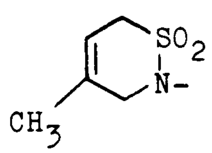 | H | $CH_3$ | $CH_3$ | CH | |
| 379 | 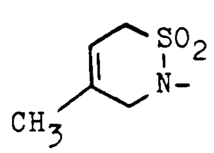 | H | $OCH_3$ | $CH_3$ | N | |

**Tabelle 1** (Fortsetzung)

| Bsp. | Q | $R^3$ | $R^5$ | $R^6$ | E | Fp |
|---|---|---|---|---|---|---|
| 380 | 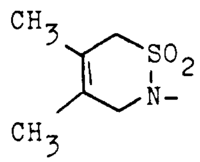 | H | $OCH_3$ | $OCH_3$ | CH | |
| 381 | 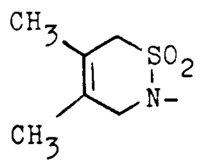 | H | $OCH_3$ | $CH_3$ | CH | |
| 382 | 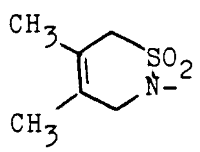 | H | $OCH_3$ | Cl | CH | |
| 383 | 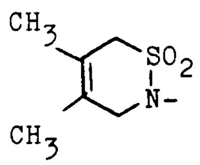 | H | $OCHF_2$ | $OCHF_2$ | CH | |
| 384 | 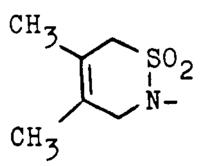 | H | $OCH_3$ | $CH_3$ | N | |
| 385 | 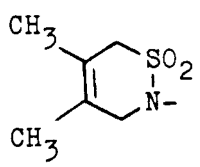 | $CH_3$ | $OCH_3$ | $CH_3$ | N | |
| 386 | 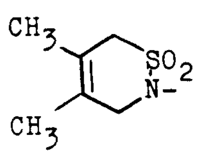 | H | $OCH_3$ | $NHCH_3$ | N | |

Tabelle 1 (Fortsetzung)

| Bsp. | Q | $R^3$ | $R^5$ | $R^6$ | E | Fp |
|---|---|---|---|---|---|---|
| 387 | 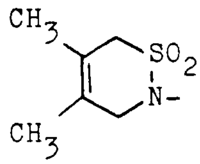 | H | $OCH_3$ | $OCH_2CF_3$ | N | |
| 388 | 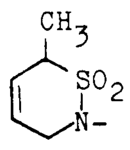 | H | $OCH_3$ | $OCH_3$ | CH | |
| 389 | 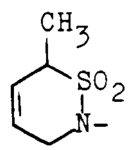 | $CH_3$ | $OCH_3$ | $OCH_3$ | CH | |
| 390 | 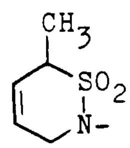 | H | $OCH_3$ | $CH_3$ | N | |
| 391 | 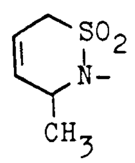 | H | $OCH_3$ | $OCH_3$ | CH | |
| 392 | 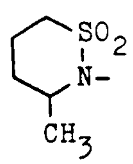 | H | $OCH_3$ | Cl | CH | |
| 393 | 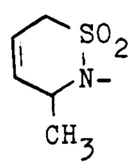 | H | $CH_3$ | $CH_3$ | CH | |
| 394 | 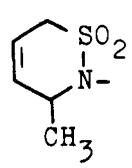 | H | $OCH_3$ | $CH_3$ | N | |

Tabelle 1 (Fortsetzung)

| Bsp. | Q | $R^3$ | $R^5$ | $R^6$ | E | Fp |
|---|---|---|---|---|---|---|
| 395 | 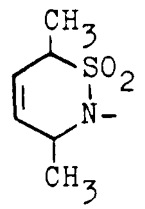 | H | $OCH_3$ | $OCH_3$ | CH | |
| 396 | 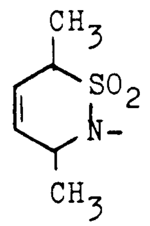 | H | $OCH_3$ | $CH_3$ | CH | |
| 397 | 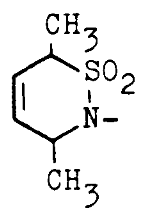 | H | $CH_3$ | $CH_3$ | CH | |
| 398 | 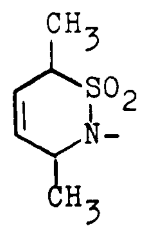 | H | $OCH_3$ | Cl | CH | |
| 399 | 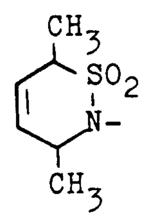 | H | $OCH_3$ | $CH_3$ | N | |

**Tabelle 2**

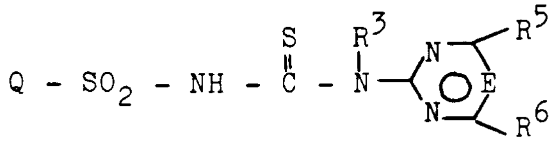

| Bsp. | Q | $R^3$ | $R^5$ | $R^6$ | E | Fp |
|---|---|---|---|---|---|---|
| 400 | 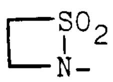 | H | $OCH_3$ | $OCH_3$ | CH | |
| 401 | 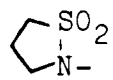 | H | $OCH_3$ | $OCH_3$ | CH | |
| 402 | 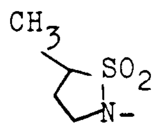 | H | $OCH_3$ | $OCH_3$ | CH | |
| 403 | 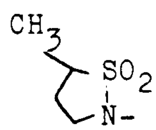 | H | $OCH_3$ | $CH_3$ | N | |
| 404 | 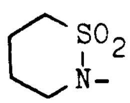 | H | $OCH_3$ | $OCH_3$ | CH | |
| 405 | 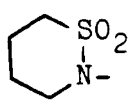 | H | $OCH_3$ | $CH_3$ | N | |
| 406 | 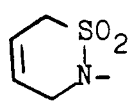 | H | $OCH_3$ | $OCH_3$ | CH | |
| 407 | 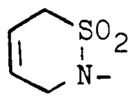 | H | $OCH_3$ | Cl | CH | |
| 408 | 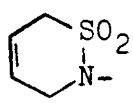 | H | $OCH_3$ | $OCH_3$ | CH | |

Tabelle 2 (Fortsetzung)

| Bsp. | Q | $R^3$ | $R^5$ | $R^6$ | E | Fp |
|---|---|---|---|---|---|---|
| 409 | 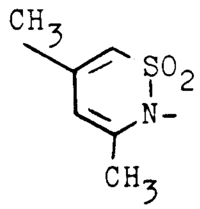 | H | $OCH_3$ | $OCH_3$ | CH | |
| 410 | 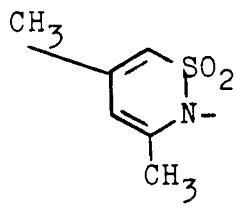 | H | $CH_3$ | $CH_3$ | CH | |
| 411 | 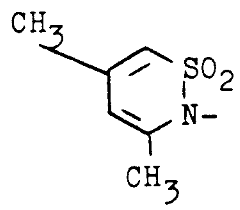 | H | $OCH_3$ | $OCH_3$ | N | |

**Biologische Beispiele**

Die Schädigung der Unkrautpflanzen bzw. die Kulturpflanzenverträglichkeit wurde gemäß einem Schlüssel bonitiert, in dem die Wirksamkeit durch Wertzahlen von 0 bis 5 ausgedrückt ist. Dabei bedeutet:

0 = ohne Wirkung
1 = 0 bis 20 % Wirkung bzw. Schaden
2 = 20 bis 40 % Wirkung bzw. Schaden
3 = 40 bis 60 % Wirkung bzw. Schaden
4 = 60 bis 80 % Wirkung bzw. Schaden
5 = 80 bis 100% Wirkung bzw. Schaden

1. Unkrautwirkung im Vorauflauf

Samen bzw. Rhizomstücke von mono- und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3 bis 4 Wochen im Vergleich zu unbehandelten Kontrollen. Wie die Boniturwerte zeigen, weisen die erfindungsgemäßen Verbindungen eine gute herbizide Vorauflaufwirksamkeit gegen ein breites Spektrum von Ungräsern und Unkräutern auf.

2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert.

Die erfindungsgemäßen Mittel weisen auch im Nachauflauf eine gute herbizide Wirksamkeit gegen ein breites Spektrum wirtschaftlich wichtiger Ungräser und Unkräuter auf.

3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt.

Ein Teil der Töpfe wurde sofort wie unter 1. beschrieben behandelt, die übrigen im Gewächshaus aufgestellt, bis die Pflanzen zwei bis drei echte Blätter entwickelt hatten und dann mit den erfindungsgemäßen Substanzen in unterschiedlichen Dosierungen, wie unter 2. beschrieben, besprüht.

Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z.B. Gerste, Weizen, Roggen, Sorghum-Hirsen, Mais oder Reis. Die Verbindungen der Formel I weisen somit eine hohe Selektivität bei Anwendung zur Bekämpfung von unerwünschtem Pflanzenwuchs in landwirtschaftlichen Kulturen auf.

Tabelle 3

| Vorauflaufwirkung der erfindungsgemäßen Verbindungen | | | | | |
|---|---|---|---|---|---|
| Produkt Nr. | Dosis kg a.i./ha | herbizide Wirkung | | | |
| | | SIA | CRS | STM | LOM |
| 2 | 0,6 | 5 | 5 | 5 | 5 |
| 3 | 0,6 | 5 | 5 | 5 | 5 |
| 6 | 0,6 | 5 | 5 | 4 | 5 |
| 18 | 0,6 | 5 | 5 | 5 | 5 |
| 30 | 0,6 | 5 | 5 | 5 | 5 |
| 52 | 0,6 | 5 | 4 | 5 | 4 |
| 101 | 0,6 | 5 | 5 | 5 | 5 |
| 102 | 0,6 | 5 | 5 | 5 | 5 |
| 143 | 0,6 | 5 | 5 | 5 | 5 |
| 220 | 0,6 | 5 | 5 | 5 | 5 |
| 221 | 0,6 | 5 | 5 | 5 | 5 |
| 227 | 0,6 | 5 | 5 | 5 | 5 |
| 256 | 0,6 | 5 | 5 | 4 | 5 |
| 306 | 0,6 | 5 | 5 | 5 | 5 |

Tabelle 4

| Nachauflaufwirkung | | | | | |
|---|---|---|---|---|---|
| Produkt Hoe-Nr. | Dosis kg a.i./ha | herbizide Wirkung | | | |
| | | SIA | CRS | STM | LOM |
| 2 | 0,6 | 5 | 5 | 5 | 5 |
| 3 | 0,6 | 5 | 5 | 5 | 5 |
| 6 | 0,6 | 5 | 5 | 4 | 5 |
| 18 | 0,6 | 5 | 5 | 5 | 5 |
| 30 | 0,6 | 4 | 5 | 5 | 5 |
| 52 | 0,6 | 5 | 5 | 5 | 5 |
| 101 | 0,6 | 5 | 5 | 5 | 5 |
| 102 | 0,6 | 5 | 5 | 5 | 5 |
| 143 | 0,6 | 5 | 5 | 5 | 5 |
| 220 | 0,6 | 5 | 5 | 5 | 5 |
| 221 | 0,6 | 5 | 5 | 5 | 5 |
| 222 | 0,6 | 5 | 5 | 5 | 5 |
| 256 | 0,6 | 5 | 5 | 5 | 5 |
| 306 | 0,6 | 5 | 5 | 5 | 5 |

Abkürzungen:
SIA = Sinapsis alba
CRS = Chrysanthemum segetum
STM = Stellaria media
LOM = Lolium multiflorum

**Wuchshemmung an Getreide**

In Schalenversuchen im Gewächshaus wurden junge Getreidepflanzen (Weizen, Gerste, Roggen) im 3-Blattstadium mit erfindungsgemäßen Verbindungen in verschiedenen Wirkstoffkonzentrationen (kg/ha) tropfnass gespritzt.

Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Es wurde außerdem die phytotoxische Wirkung der Verbindungen beobachtet, wobei 100 % den Stillstand des Wachstums und 0 % ein Wachstum entsprechend den unbehandelten Kontrollpflanzen bedeuten. Es zeigte sich, daß die Verbindungen sehr gute wachstumsregulierende Eigenschaften besitzen. Die Ergebnisse sind in der nachfolgenden Tabelle zusammengestellt.

Tabelle 5

| Verbindungen nach Bsp.-Nr. | Anwendungskonz. kg/ha | Wuchshemmung (%) | | | Phytotox. Wirkung |
|---|---|---|---|---|---|
| | | Weizen | Gerste | Roggen | |
| 3 | 0.62<br>0.31 | 25<br>18 | 24<br>17 | 43<br>29 | keine<br>Schäden |
| 101 | 0.62<br>0.31 | 19<br>15 | 22<br>17 | 35<br>29 | keine<br>Schäden |

**Patentansprüche**

**Patentansprüche für folgende Vertragsstaaten : AT, BE, CH, DE, FR, GB, IT, LI, NL**

1. Verbindungen der Formel I oder deren Salze

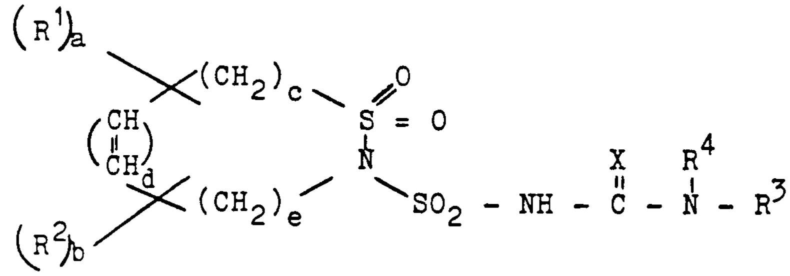

(I),

worin

| | |
|---|---|
| $R^1$ und $R^2$ | unabhängig voneinander Wasserstoff, Halogen, $(C_1$-$C_8)$-Alkyl, $(C_2$-$C_8)$-Alkenyl, $(C_2$-$C_8)$-Alkinyl oder $(C_1$-$C_8)$-Alkoxy, wobei diese Reste gegebenenfalls ein- oder mehrfach durch Halogen oder ein- oder zweifach durch $(C_1$-$C_4)$-Alkoxy oder $(C_1$-$C_4)$-Alkylthio substituiert sein können; $-(CH_2)_n$-$COOR^{11}$, wobei n eine Zahl zwischen 0 und 2 bedeutet, |
| $R^3$ | Wasserstoff; $(C_1$-$C_8)$-Alkyl; $(C_2$-$C_8)$-Alkenyl oder $(C_2$-$C_8)$-Alkinyl, |
| $R^4$ | einen heterocyclischen Rest der Formeln |

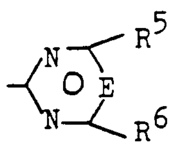

oder

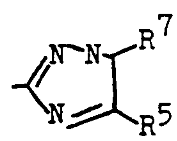

| | |
|---|---|
| | wobei E = CH oder N ist, |
| $R^5$, $R^6$ | unabhängig voneinander Wasserstoff; Halogen; $(C_1$-$C_4)$-Alkyl oder $(C_1$-$C_4)$-Alkoxy, die beide gegebenenfalls ein- oder mehrfach halogeniert sein können; Di-[$(C_1$-$C_4)$-alkoxy]-$(C_1$-$C_2)$-alkyl; $(C_3$-$C_6)$Cycloalkyl, $-OCHR^8COOR^9$; $-NR^9R^{10}$ oder $(C_1$-$C_4)$-Alkylthio, |
| $R^7$ | $(C_1$-$C_4)$-Alkyl, |
| $R^8$ | Wasserstoff oder $(C_1$-$C_4)$-Alkyl und |
| $R^9$, $R^{10}$ | unabhängig voneinander Wasserstoff; $(C_1$-$C_4)$-Alkyl; $(C_2$-$C_4)$-Alkenyl oder $(C_2$-$C_4)$-Alkinyl |
| $R^{11}$ | Wasserstoff, $(C_1$-$C_8)$-Alkyl, $(C_2$-$C_4)$-Alkenyl, $(C_2$-$C_4)$-Alkinyl, welche gegebenenfalls durch Halogen oder $(C_1$-$C_4)$-Alkoxyreste ein- oder mehrfach substituiert sein können, |
| X | Sauerstoff oder Schwefel und |
| a, b, c, d und e | unabhängig voneinander die Zahl 0, 1 oder 2 bedeuten, mit der Maßgabe, daß die Summe von c c + d + e größer oder gleich 2 ist. |

2. Verbindungen der Formel I von Anspruch 1 worin $R^1$, $R^2$ unabhängig voneinander Wasserstoff, $(C_1$-$C_4)$-Alkyl, das wie oben beschrieben substituiert ist oder Halogen; a, b unabhängig voneinander die Zahlen 0, 1 oder 2 bedeuten, jedoch mit der Maßgabe, daß c + d + e ≧ 2 und ≦ 4 ist, $R^3$ Wasserstoff, $(C_1$-$C_4)$-Alkyl oder Allyl; $R^4$ einen Rest der Formel

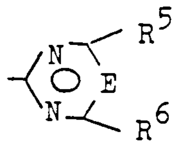

und $R^5$ und $R^6$ unabhängig voneinander Halogen, $(C_1\text{-}C_4)$-Alkyl oder $(C_1\text{-}C_4)$-Alkoxy, die beide durch Halogen substituiert sein können, bedeuten und X für Sauerstoff steht.

**3.** Verbindungen nach Anspruch 1 oder 2, dadurch gekennzeichnet, daß
$R^1$ und $R^2$ unabhängig Voneinander Wasserstoff oder $(C_1\text{-}C_4)$-Alkyl, $R^3$ Wasserstoff, a + b = 0, 1 oder 2, d = 0 und c + e = 3 oder 4 bedeuten.

**4.** Verbindungen nach einem der Ansprüche 1 bis 3, dadurch gekennzeichnet, daß $R^5$ und $R^6$ unabhängig voneinander Chlor, Brom, $(C_1\text{-}C_4)$-Alkyl, $(C_1\text{-}C_4)$-Alkoxy, $OCF_2H$, $OCH_2CF_3$ oder $CF_3$ bedeuten.

**5.** Verbindungen nach einem der Ansprüche 1 bis 4, dadurch gekennzeichnet, daß $R^5$ und $R^6$ unabhängig voneinander $CH_3$ oder $OCH_3$ bedeuten.

**6.** Verfahren zur Herstellung der Verbindungen der Formel I nach einem der Ansprüche 1 bis 5 oder deren Salze, dadurch gekennzeichnet, daß man
a) eine Verbindung der Formel (II)

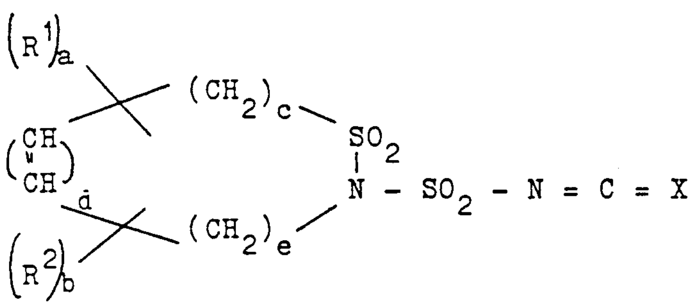

(II)

mit einer Verbindung der Formel (III)

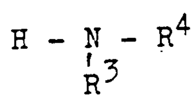

(III)

umsetzt, oder
b) für X = Sauerstoff eine Verbindung der Formel (IV)

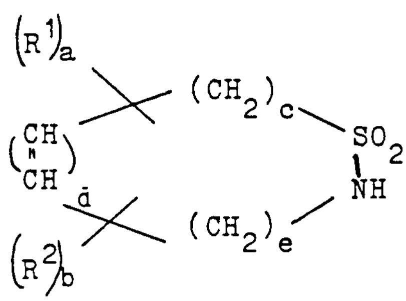

(IV)

mit einem Chlorsulfonylharnstoff der Formel (V)

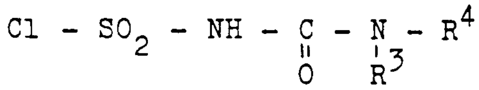 (V)

umsetzt und die erhaltene Verbindung gegebenenfalls in ihr Salz überführt.

**7.** Herbizide oder pflanzenwachstumsregulierende Mittel, dadurch gekennzeichnet, daß sie eine Verbindung der Formel I nach einem der Ansprüche 1 bis 5 oder deren Salz neben geeigneten Formulierungshilfsmitteln enthalten.

**8.** Verwendung der Verbindungen der Formel I nach einem der Ansprüche 1 bis 5 oder deren Salze als Herbizide oder Pflanzenwachstumsregulatoren.

**9.** Verfahren zur Bekämpfung von unerwünschtem Pflanzenwuchs, dadurch gekennzeichnet, daß man auf die Pflanzen oder die Kulturflächen eine wirksame Menge einer Verbindung der Formel I oder deren Salznach einem der Ansprüche 1 bis 5 appliziert.

**10.** Verfahren zur Pflanzenwachstumsregulierung, dadurch gekennzeichnet, daß man auf die Pflanzen oder deren Anbaufläche eine wirksame Menge einer Verbindung nach einem der Ansprüche 1 bis 5 oder deren Salz appliziert.

**Patentansprüche für folgenden Vertragsstaat : ES**

**1.** Verfahren zur Herstellung von Verbindungen der Formel I oder deren Salzen

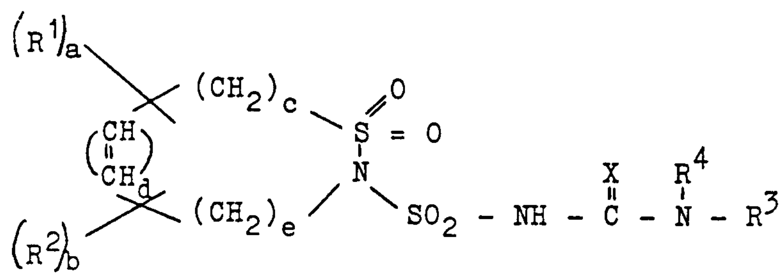 (I),

worin

$R^1$ und $R^2$ unabhängig voneinander Wasserstoff, Halogen, $(C_1\text{-}C_8)$-Alkyl, $(C_2\text{-}C_8)$-Alkenyl, $(C_2\text{-}C_8)$-Alkinyl oder $(C_1\text{-}C_8)$-Alkoxy, wobei diese Reste gegebenenfalls ein- oder mehrfach durch Halogen oder ein- oder zweifach durch $(C_1\text{-}C_4)$-Alkoxy oder $(C_1\text{-}C_4)$-Alkylthio substituiert sein können; $-(CH_2)_n\text{-}COOR^{11}$, wobei n eine Zahl zwischen 0 und 2 bedeutet,

$R^3$ Wasserstoff; $(C_1\text{-}C_8)$-Alkyl; $(C_2\text{-}C_8)$-Alkenyl oder $(C_2\text{-}C_8)$-Alkinyl,

$R^4$ einen heterocyclischen Rest der Formeln

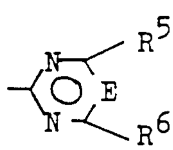 oder 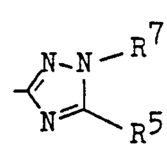

wobei E = CH oder N ist,

$R^5$, $R^6$ unabhängig voneinander Wasserstoff; Halogen; $(C_1\text{-}C_4)$-Alkyl oder $(C_1\text{-}C_4)$-Alkoxy, die beide gegebenenfalls ein- oder mehrfach halogeniert sein können; Di-$[(C_1\text{-}C_4)$-alkoxy]-$(C_1\text{-}C_2)$-alkyl; $(C_3\text{-}C_6)$Cycloalkyl, $-OCHR^8COOR^9$; $-NR^9R^{10}$ oder $(C_1\text{-}C_4)$-Alkylthio,

| | |
|---|---|
| $R^7$ | $(C_1\text{-}C_4)$-Alkyl, |
| $R^8$ | Wasserstoff oder $(C_1\text{-}C_4)$-Alkyl und |
| $R^9$, $R^{10}$ | unabhängig voneinander Wasserstoff; $(C_1\text{-}C_4)$-Alkyl; $(C_2\text{-}C_4)$-Alkenyl oder $(C_2\text{-}C_4)$-Alkinyl |
| $R^{11}$ | Wasserstoff, $(C_1\text{-}C_8)$-Alkyl, $(C_2\text{-}C_4)$-Alkenyl, $(C_2\text{-}C_4)$-Alkinyl, welche gegebenenfalls durch Halogen oder $(C_1\text{-}C_4)$-Alkoxyreste ein- oder mehrfach substituiert sein können, |
| X | Sauerstoff oder Schwefel und |
| a, b, c , d und e | unabhängig voneinander die Zahl 0, 1 oder 2 bedeuten, mit der Maßgabe, daß die Summe von c c + d + e größer oder gleich 2 ist, dadurch gekennzeichnet, daß man |

a) eine Verbindung der Formel (II)

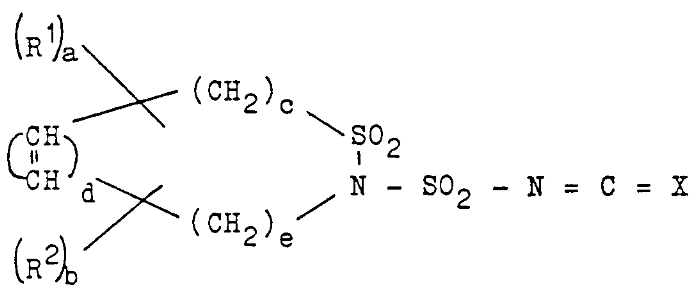

(II)

mit einer Verbindung der Formel (III)

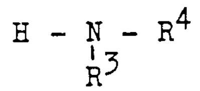

(III)

umsetzt, oder

b) für X = Sauerstoff eine Verbindung der Formel (IV)

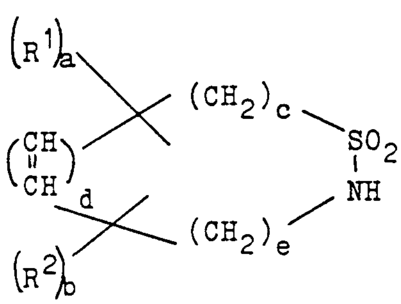

(IV)

mit einem Chlorsulfonylharnstoff der Formel (V)

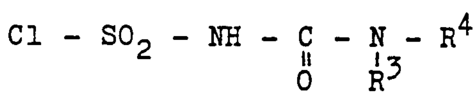

(V)

umsetzt und die erhaltene Verbindung gegebenenfalls in ihr Salz überführt.

**2.** Verfahren gemäß Anspruch 1, dadurch gekennzeichnet, daß in den Formeln I - V $R^1$, $R^2$ unabhängig voneinander Wasserstoff, $(C_1\text{-}C_4)$-Alkyl, das wie oben beschrieben substituiert ist oder Halogen; a, b, c, d, e unabhängig voneinander die Zahlen 0, 1 oder 2 bedeuten, jedoch mit der Maßgabe, daß c + d + e $\geqq$ 2 und $\leqq$ 4 ist, $R^3$ Wasserstoff, $(C_1\text{-}C_4)$-Alkyl oder Allyl; $R^4$ einen Rest der Formel

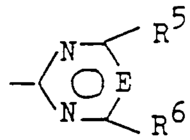

und $R^5$ und $R^6$ unabhängig voneinander Halogen, $(C_1-C_4)$-Alkyl oder $(C_1-C_4)$-Alkoxy, die beide durch Halogen substituiert sein können, bedeuten und X für Sauerstoff steht.

3. Verfahren nach Anspruch 1 oder 2, dadurch gekennzeichnet, $R^1$ und $R^2$ unabhängig voneinander Wasserstoff oder $(C_1-C_4)$-Alkyl, $R^3$ Wasserstoff, a + b = 0, 1 oder 2, d = 0 und c + e = 3 oder 4 bedeuten.

4. Verfahren nach einem der Ansprüche 1 bis 3, dadurch gekennzeichnet, daß $R^5$ und $R^6$ unabhängig voneinander Chlor, Brom, $(C_1-C_4)$-Alkyl, $(C_1-C_4)$-Alkoxy, $OCF_2H$, $OCH_2CF_3$ oder $CF_3$ bedeuten.

5. Verfahren nach einem der Ansprüche 1 bis 4, dadurch gekennzeichnet, daß $R^5$ und $R^6$ unabhängig voneinander $CH_3$ oder $OCH_3$ bedeuten.

6. Verwendung der Verbindungen der Formel I nach einem der Ansprüche 1 bis 5 oder deren Salze als Herbizide oder Pflanzenwachstumsregulatoren.

7. Verfahren zur Bekämpfung von unerwünschtem Pflanzenwuchs oder zur Wachstumsregulierung von Pflanzen, dadurch gekennzeichnet, daß man auf die Pflanzen oder die Kulturböden eine wirksame Menge einer Verbindung der Formel I nach einem der Ansprüche 1 bis 5 oder deren Salz appliziert.

**Claims**

**Claims for the following Contracting States : AT, BE, CH, DE, FR, GB, IT, LI, NL**

1. A compound of the formula I or a salt thereof

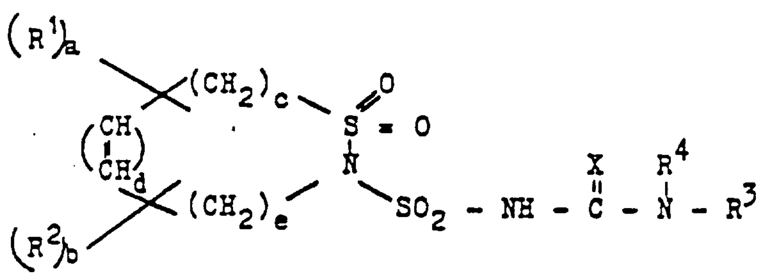

(I),

in which

| | |
|---|---|
| $R^1$ and $R^2$ | independently of one another denote hydrogen, halogen, $(C_1-C_8)$-alkyl, $(C_2-C_8)$-alkenyl, $(C_2-C_8)$-alkynyl or $(C_1-C_8)$-alkoxy, it being possible for these radicals to be optionally substituted once or more than once by halogen or to be substituted once or twice by $(C_1-C_4)$-alkoxy or $(C_1-C_4)$-alkylthio; or denote $-(CH_2)_n-COOR^{11}$, where n denotes a number between 0 and 2, |
| $R^3$ | denotes hydrogen; $(C_1-C_8)$-alkyl; $(C_2-C_8)$-alkenyl or $(C_2-C_8)$-alkynyl, |
| $R^4$ | denotes a heterocyclic radical of the formula |

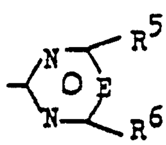

or

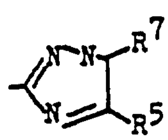

where E is CH or N,

$R^5$ and $R^6$ independently of one another denote hydrogen; halogen; $(C_1-C_4)$alkyl or $(C_1-C_4)$-alkoxy, both or which can optionally be halogenated once of more than once; di-$[(C_1-C_4)$-alkoxy$]$-$(C_1-C_2)$-alkyl; $(C_3-C_6)$-cycloalkyl, $-OCHR^8COOR^9$; $-NR^9R^{10}$ or $(C_1-C_4)$-alkylthio,

$R^7$ denotes $(C_1-C_4)$-alkyl,

$R^8$ denotes hydrogen or $(C_1-C_4)$-alkyl and

$R^9$ and $R^{10}$ independently of one another denote hydrogen; $(C_1-C_4)$-alkyl; $(C_2-C_4)$-alkenyl or $(C_2-C_4)$-alkynyl,

$R^{11}$ denotes hydrogen, $(C_1-C_8)$-alkyl, $(C_2-C_4)$-alkenyl or $(C_2-C_4)$-alkynyl, each of which can optionally be substituted once or more than once by halogen or $(C_1-C_4)$-alkoxy radicals,

X denotes oxygen or sulfur and

a, b, c, d and e independently of one another denote the number 0, 1 or 2, with the proviso that the total of c + d + e is larger than, or equals, 2.

**2.** A compound of the formula I as claimed in claim 1 wherein $R^1$ and $R^2$ independently of one another denote hydrogen, $(C_1-C_4)$-alkyl which is substituted as described above, or halogen; a and b independently of one another denote the numbers 0, 1 or 2, but with the proviso that $c + d + e \geqq 2$ and $\leqq 4$, $R^3$ denotes hydrogen, $(C_1-C_4)$-alkyl or allyl; $R^4$ denotes a radical of the formula

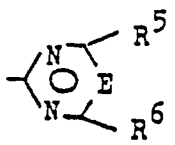

and $R^5$ and $R^6$ independently of one another denote halogen, $(C_1-C_4)$-alkyl or $(C_1-C_4)$-alkoxy, both of which can be substituted by halogen, and

X stands for oxygen.

**3.** A compound as claimed in claim 1 or 2, wherein

$R^1$ and $R^2$ independently of one another denote hydrogen or $(C_1-C_4)$-alkyl,

$R^3$ denotes hydrogen, $a + b = 0, 1$ or $2$, $d = 0$ and $c + e = 3$ or $4$.

**4.** A compound as claimed in any one of claims 1 to 3, wherein $R^5$ and $R^6$ independently of one another denote chlorine, bromine, $(C_1-C_4)$-alkyl,$(C_1-C_4)$-alkoxy, $OCF_2H$, $OCH_2CF_3$ or $CF_3$.

**5.** A compound as claimed in any one of claims 1 to 4, wherein $R^5$ and $R^6$ independently of one another denote $CH_3$ or $OCH_3$.

**6.** A process for the preparation of a compound of the formula I as claimed in any one of claims 1 to 5 or a salt thereof, which comprises

a) reacting a compound of the formula (II)

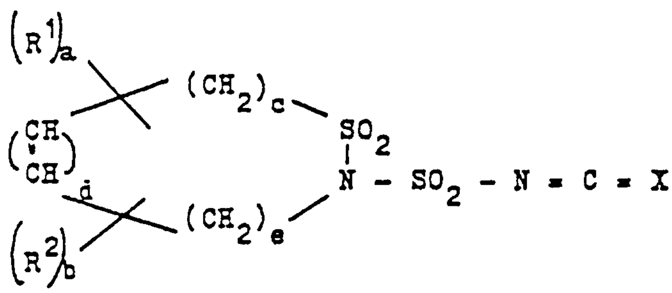

(II)

with a compound of the formula (III)

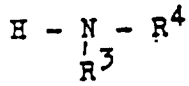 (III)

or

(b) when X is oxygen, reacting a compound of the formula (IV)

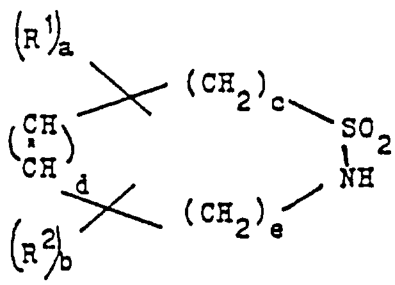 (IV)

with a chlorosulfonylurea of the formula (V)

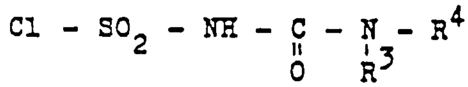 (V)

and, if appropriate, converting the resulting compound into a salt thereof.

**7.** A herbicide or plant growth-regulating agent containing a compound of the formula I as claimed in any one of claims 1 to 5 or a salt thereof, in addition to suitable formulation auxiliaries.

**8.** The use of a compound of the formula I as claimed in any one of claims 1 to 5 or a salt thereof as a herbicide or plant growth regulator.

**9.** A method of controlling undesired plant growth, wherein an effective amount of a compound of the formula I or a salt thereof as claimed in any one of claims 1 to 5 is applied to the plants or to the areas where they are grown.

**10.** A method of regulating plant growth, wherein an effective amount of a compound as claimed in any one of claims 1 to 5 or a salt thereof is applied to the plants or to the area where they are grown.

**Claims for the following Contracting State : ES**

**1.** A process for the preparation of a compound of the formula I or a salt thereof

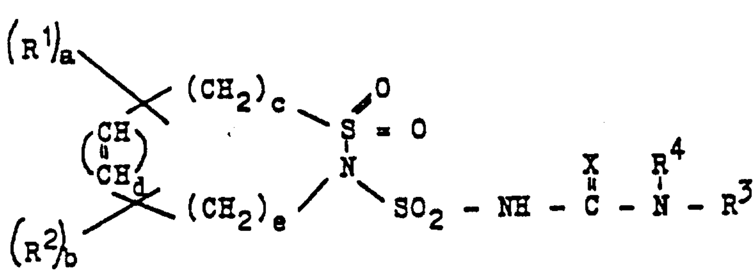 (I),

in which

$R^1$ and $R^2$ independently of one another denote hydrogen, halogen, $(C_1-C_8)$-alkyl, $(C_2-C_8)$-alkenyl, $(C_2-C_8)$-alkynyl or $(C_1-C_8)$-alkoxy, it being possible for these radicals to be optionally substituted once or more than once by halogen or to be substituted once or twice by $(C_1-C_4)$-alkoxy or $(C_1-C_4)$-alkylthio; or denote $-(CH_2)_n-COOR^{11}$, where n denotes a number between 0 and 2,

$R^3$ denotes hydrogen; $(C_1-C_8)$-alkyl; $(C_2-C_8)$-alkenyl or $(C_2-C_8)$-alkynyl,

$R^4$ denotes a heterocyclic radical of the formula

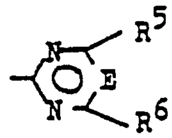

or

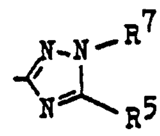

where E is CH or N,

$R^5$ and $R^6$ independently of one another denote hydrogen; halogen; $(C_1-C_4)$-alkyl or $(C_1-C_4)$-alkoxy, both of which can optionally be halogenated once of more than once; di-[$(C_1-C_4)$-alkoxy]-$(C_1-C_2)$-alkyl; $(C_3-C_6)$-cycloalkyl, $-OCHR^8COOR^9$; $-NR^9R^{10}$ or $(C_1-C_4)$-alkylthio,

$R^7$ denotes $(C_1-C_4)$-alkyl,

$R^8$ denotes hydrogen or $(C_1-C_4)$-alkyl and

$R^9$ and $R^{10}$ independently of one another denote hydrogen; $(C_1-C_4)$-alkyl; $(C_2-C_4)$-alkenyl or $(C_2-C_4)$-alkynyl,

$R^{11}$ denotes hydrogen, $(C_1-C_8)$-alkyl, $(C_2-C_4)$-alkenyl or $(C_2-C_4)$-alkynyl, each of which can optionally be substituted once or more than once by halogen or $(C_1-C_4)$-alkoxy radicals,

X denotes oxygen or sulfur and

a, b, c, d and e independently of one another denote the number 0, 1 or 2, with the proviso that the total of c + d + e is larger than, or equals, 2, which process comprises

a) reacting a compound of the formula (II)

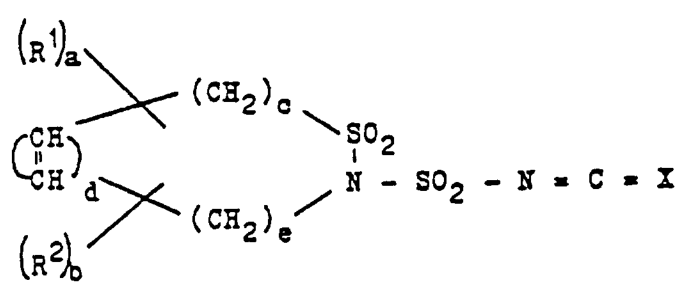

(II)

with a compound of the formula (III)

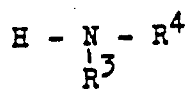

(III)

or

b) when X is oxygen, reacting a compound of the formula (IV)

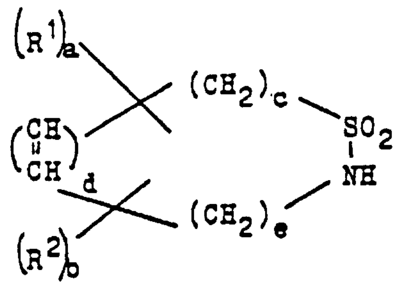

(IV)

with a chlorosulfonylurea of the formula (V)

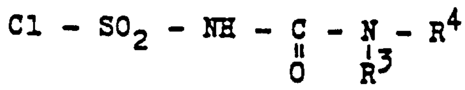

(V)

and, if appropriate, converting the resulting compound into a salt thereof.

**2.** The process as claimed in claim 1, wherein, in the formulae I-V, $R^1$ and $R^2$ independently of one another denote hydrogen, $(C_1-C_4)$-alkyl which is substituted as described above, or halogen; a, b, c, d and e independently of one another denote the numbers 0, 1 or 2, but with the proviso that $c + d + e \geqq 2$ and $\leqq 4$, $R^3$ denotes hydrogen, $(C_1-C_4)$-alkyl or allyl; $R^4$ denotes a radical of the formula

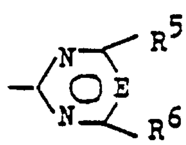

and $R^5$ and $R^6$ independently of one another denote halogen, $(C_1-C_4)$-alkyl or $(C_1-C_4)$-alkoxy, both of which can be substituted by halogen, and X stands for oxygen.

**3.** The process as claimed in claim 1 or 2, wherein

| | |
|---|---|
| $R^1$ and $R^2$ | independently of one another denote hydrogen or $(C_1-C_4)$-alkyl, |
| $R^3$ | denotes hydrogen, $a + b = 0$, 1 or 2, $d = O$ and $c + e = 3$ or 4. |

**4.** The process as claimed in any one of claims 1 to 3, wherein $R^5$ and $R^6$ independently of one another denote chlorine, bromine, $(C_1-C_4)$-alkyl,$(C_1-C_4)$-alkoxy, $OCF_2H$, $OCH_2CF_3$ or $CF_3$.

**5.** The process as claimed in any one of claims 1 to 4, wherein $R^5$ and $R^6$ independently of one another denote $CH_3$ or $OCH_3$.

**6.** The use of a compound of the formula I as claimed in any one of claims 1 to 5 or a salt thereof as a herbicide or plant growth regulator.

**7.** A method of controlling undesired plant growth or regulating plant growth, wherein an effective amount of a compound of the formula I as claimed in any one of claims 1 to 5 or a salt thereof is applied to the plants or the soils on which they are grown.

**Revendications**

**Revendications pour les Etats contractants suivants : AT, BE, CH, DE, FR, GB, IT, LI, NL**

**1.** Composés de formule I ou leurs sels

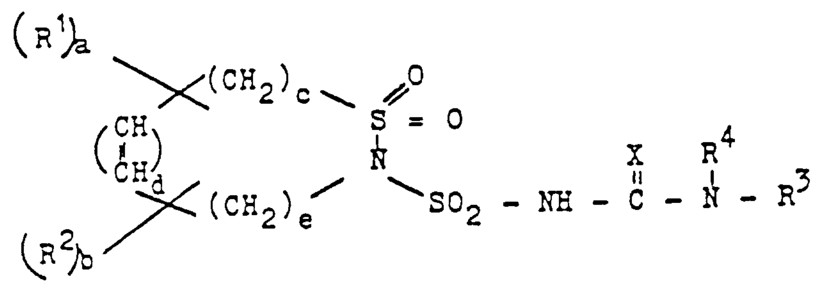

(I),

où

$R^1$ et $R^2$, indépendamment l'un de l'autre, représentent l'hydrogène, un halogène, un alkyle en ($C_1$-$C_8$), un alcényle en ($C_2$-$C_8$), un alcynyle en ($C_2$-$C_8$) ou un alcoxy en ($C_1$-$C_8$), ces restes pouvant être éventuellement substitués une ou plusieurs fois par un halogène ou une ou deux fois par un alcoxy en ($C_1$-$C_4$) ou par un alkylthio en ($C_1$-$C_4$); $-(CH_2)_nCOOR^{11}$, où n représente un nombre entre 0 et 2;

$R^3$ représente l'hydrogène; un alkyle en ($C_1$-$C_8$); un alcényle en ($C_2$-$C_8$) ou un alcynyle en ($C_2$-$C_8$),

$R^4$ représente un reste hétérocyclique des formules

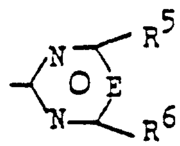

ou

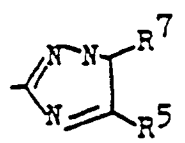

où E = CH ou N;

$R^5$, $R^6$, indépendamment l'un de l'autre, représentent l'hydrogène; un halogène; un alkyle en ($C_1$-$C_4$) ou un alcoxy en ($C_1$-$C_4$), qui tous les deux peuvent être éventuellement halogénés une ou plusieurs fois; un di-[alcoxy en ($C_1$-$C_4$)]-alkyle en ($C_1$-$C_2$); un cycloalkyle en ($C_3$-$C_6$); $-OCHR^8COOR^9$; $-NR^9R^{10}$ ou un alkylthio en ($C_1$-$C_4$),

$R^7$ représente un alkyle en ($C_1$-$C_4$),

$R^8$ représente l'hydrogène ou un alkyle en ($C_1$-$C_4$) et

$R^9$, $R^{10}$, indépendamment l'un de l'autre, représentent l'hydrogène; un alkyle en ($C_1$-$C_4$), un alcényle en ($C_2$-$C_4$) ou un alcynyle en ($C_2$-$C_4$),

$R^{11}$ représente l'hydrogène, un alkyle en ($C_1$-$C_8$), un alcényle en ($C_2$-$C_4$), un alcynyle en ($C_2$-$C_4$), qui peuvent être éventuellement substitués une ou plusieurs fois par un halogène ou un reste alcoxy en ($C_1$-$C_4$),

X représente l'oxygène ou le soufre, et

a, b, c, d et e, independamment les uns des autres, représentent le nombre 0, 1 ou 2, à la condition que la somme de c + d + e soit supérieure ou égale à 2.

**2.** Composés de formule I selon la revendication 1, où $R^1$, $R^2$, indépendamment l'un de l'autre, représentent l'hydrogène, un alkyle en ($C_1$-$C_4$), qui est substitué comme décrit plus haut, ou un halogène;

a, b, indépendamment l'un de l'autre, représentent les nombres 0, 1 ou 2, cependant à la condition que $c + d + e \geqq 2$ et $\leqq 4$,

$R^3$ représente l'hydrogène, un alkyle en ($C_1$-$C_4$) ou l' allyle;

$R^4$ représente un reste de formule

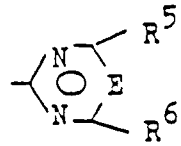

et $R^5$ et $R^6$, indépendamment l'un de l'autre, représentent un halogène, un alkyle en ($C_1$-$C_4$) ou un alcoxy en ($C_1$-$C_4$), qui peuvent être tous les deux substitués par un halogène, et X représente l'oxygène.

**3.** Composés selon la revendication 1 ou 2, caractérisés en ce que
$R^1$ et $R^2$, indépendamment l'un de l'autre, représentent l' hydrogène ou un alkyle en ($C_1$-$C_4$), $R^3$ représente l'hydrogène, a + b = 0, 1 ou 2, d = 0 et c + e = 3 ou 4.

**4.** Composés selon l'une des revendications 1 à 3, caractérisés en ce que $R^5$ et $R^6$ , indépendamment l'un de l' autre, représentent le chlore, le brome, un alkyle en ($C_1$-$C_4$), un alcoxy en ($C_1$-$C_4$), $OCF_2H$, $OCH_2CF_3$ ou $CF_3$.

**5.** Composés selon l'une des revendications 1 à 4, caractérisés en ce que $R^5$ et $R^6$, indépendamment l'un de l'autre, représentent $CH_3$ ou $OCH_3$.

**6.** Procédé pour préparer les composés de formule I selon l'une des revendications 1 à 5, ou leurs sels, caractérisé par les point suivants :
a) on fait réagir un composé de formule (II)

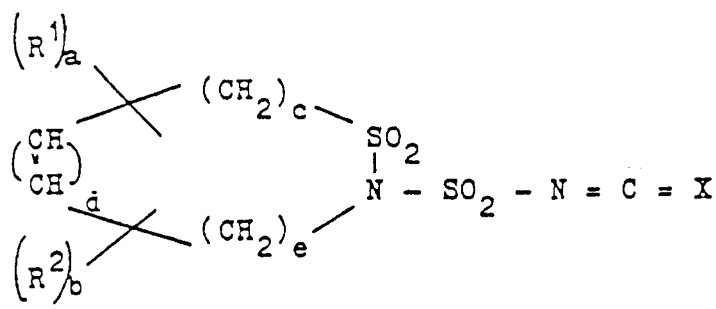

(II)

avec un composé de formule (III)

$$H - N(R^3) - R^4 \qquad (III)$$

ou

b) pour X = l'oxygène, on fait réagir un composé de formule (IV)

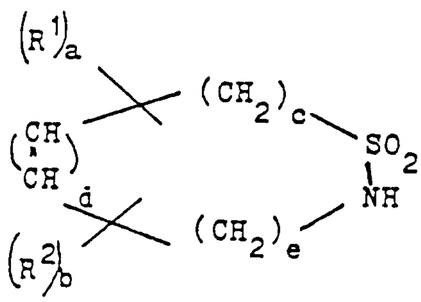

(IV)

avec une chlorosulfonylurée de formule (V)

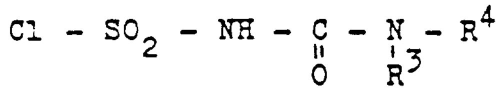

(V)

et on transforme éventuellement le composé obtenu en son sel.

**7.** Herbicides ou agents permettant de régler la croissance des plantes, caractérisés en ce qu'ils contiennent un composé de formule I selon l'une des revendications 1 à 5, ou son sel, en plus d'additifs de formulation appropriés.

**8.** Utilisation des composés de formule I selon l'une des revendications 1 à 5, ou leurs sels, comme herbicides ou comme agents permettant de régler la croissance des plantes.

**9.** Procédé pour lutter contre la croissance de plantes indésirables, caractérisé en ce que l'on applique sur les plantes ou sur les surfaces de culture une quantité efficace d'un composé de formule I ou de son sel, selon l'une des revendications 1 à 5.

**10.** Procédé pour régler la croissance des plantes, caractérisé en ce que l'on applique sur les plantes ou sur leurs terres de culture, une quantité efficace d'un composé selon l'une des revendications 1 à 5, ou de son sel.

**Revendications pour l'Etat contractant suivant : ES**

**1.** Procédé pour préparer des composés de formule I ou leurs sels

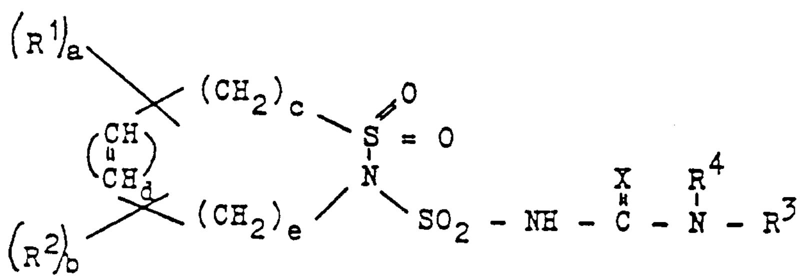

(I),

où

$R^1$ et $R^2$, indépendamment l'un de l'autre, représentent l'hydrogène, un halogène, un alkyle en ($C_1$-$C_8$), un alcényle en ($C_2$-$C_8$), un alcynyle en ($C_2$-$C_8$) ou un alcoxy en ($C_1$-$C_8$), ces restes pouvant être

éventuellement substitués une ou plusieurs fois par un halogène ou une ou deux fois par un alcoxy en ($C_1$-$C_4$) ou par un alkylthio en ($C_1$-$C_4$); $-(CH_2)_n-COOR^{11}$, où n représente un nombre entre 0 et 2,
$R^3$ représente l'hydrogène; un alkyle en ($C_1$-$C_8$); un alcényle en ($C_2$-$C_8$) ou un alcynyle en ($C_2$-$C_8$),
$R^4$ représente un reste hétérocyclique des formules

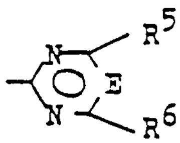

ou

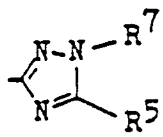

où E = CH ou N,
$R^5$ et $R^6$, indépendamment l'un de l'autre, représentent l'hydrogène; un halogène; un alkyle en ($C_1$-$C_4$) ou un alcoxy en ($C_1$-$C_4$), qui tous les deux peuvent être éventuellement halogénés une ou plusieurs fois; un di-[alcoxy en ($C_1$-$C_4$)]-alkyle en ($C_1$-$C_2$); un cycloalkyle en ($C_3$-$C_6$), $-OCHR^8COOR^9$; $-NR^9R^{10}$ ou un alkylthio en ($C_1$-$C_4$),
$R^7$ représente un alkyle en ($C_1$-$C_4$),
$R^8$ représente l'hydrogène ou un alkyle en ($C_1$-$C_4$) et
$R^9$, $R^{10}$, indépendamment l'un de l'autre, représentent l'hydrogène; un alkyle en ($C_1$-$C_4$); un alcényle en ($C_2$-$C_4$) ou un alcynyle en ($C_2$-$C_4$),
$R^{11}$ représente l'hydrogène, un alkyle en ($C_1$-$C_8$), un alcényle en ($C_2$-$C_4$), un alcynyle en ($C_2$-$C_4$), qui peuvent être éventuellement substitués une ou plusieurs fois par un halogène ou un reste alcoxy en ($C_1$-$C_4$),
X représente l'oxygène ou le soufre, et
a, b, c, d et e, indépendamment les uns des autres, représentent le nombre 0, 1 ou 2, à la condition que la somme c + d + e soit supérieure ou égale à 2, caractérisé par les points suivants :

a) on fait réagir un composé de formule (II)

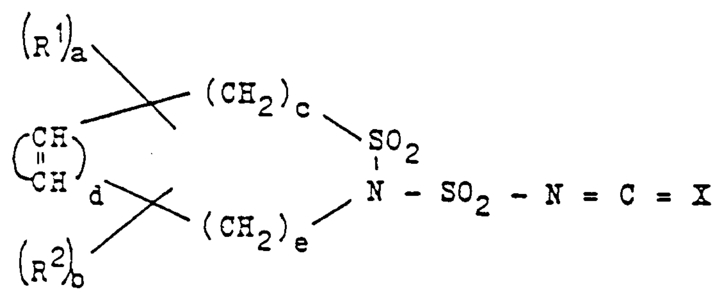

(II)

avec un composé de formule (III)

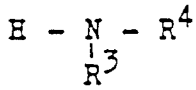

(III)

ou,

b) pour X = l'oxygène, on fait réagir un composé de formule (IV)

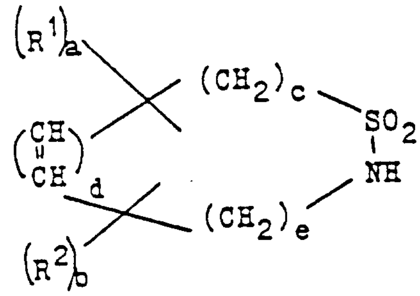

(IV)

avec une chlorosulfonylurée de formule (V)

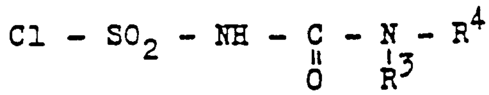

(V)

et on transforme éventuellement le composé obtenu en son sel.

**2.** Procédé selon la revendication 1, caractérisé en ce que dans les formules I à V $R^1$, $R^2$, indépendamment l'un de l'autre, représentent l'hydrogène, un alkyle en ($C_1$-$C_4$),qui peut être substitué comme décrit plus haut, ou un halogène; a, b, c, d, e, indépendamment les uns des autres, représentent les nombres 0, 1 ou 2, cependant avec la condition que $c + d + e \geqq 2$ et $\leqq 4$,
$R^3$ représente l'hydrogène, un alkyle en ($C_1$-$C_4$) ou l'allyle;
$R^4$ est un reste de formule

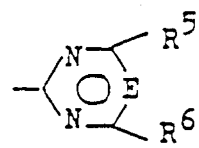

et $R^5$ et $R^6$, indépendamment l'un de l'autre, représentent un halogène, un alkyle en ($C_1$-$C_4$) ou un alcoxy en ($C_1$-$C_4$), qui peuvent être tous les deux substitués par un halogène,
et X représente l'oxygène.

**3.** Procédé selon la revendication 1 ou 2, caractérisé en ce que $R^1$ et $R^2$, indépendamment l'un de l'autre, représentent l'hydrogène ou un alkyle en ($C_1$-$C_4$), $R^3$ représente l'hydrogène, $a + b = 0$, 1 ou 2, $d = 0$ et $c + e = 3$ ou 4.

**4.** Procédé selon l'une des revendications 1 à 3, caractérisé en ce que $R^5$ et $R^6$, indépendamment l'un de l'autre, représentent le chlore, le brome, un alkyle en ($C_1$-$C_4$),un alcoxy en ($C_1$-$C_4$), $OCF_2H$, $OCH_2CF_3$, ou $CF_3$.

**5.** Procédé selon l'une des revendications 1 à 4, caractérisé en ce que $R^5$ et $R^6$, indépendamment l'un de l'autre, représentent $CH_3$ ou $OCH_3$.

**6.** Utilisation des composés de formule I selon l'une des revendications 1 à 5, ou de leurs sels, comme herbicides ou comme agents permettant de régler la croissance des plantes.

**7.** Procédé pour lutter contre la croissance de plantes indésirables ou pour régler la croissance des plantes, caractérisé en ce que l'on applique sur les plantes ou sur les sols de culture une quantité

efficace d'un composé de formule I selon l'une des revendications 1 à 5, ou de son sel.